# EUROPÄISCHE PATENTSCHRIFT

(11) **EP 1 926 726 B1**
(45) Veröffentlichungstag und Bekanntmachung des Hinweises auf die Patenterteilung: **02.05.2012**
(21) Anmeldenummer: 06774768.3
(22) Anmeldetag: 06.09.2006
(51) Int. Cl.: C07D 403/14, A61K 31/409

(54) **NICHTFLUORESZIERENDE CHLOROPHYLLKATABOLITEN**
NON-FLUORESCENT CHLOROPHYLL CATABOLITES
CATABOLITES DE CHLOROPHYLLE NON FLUORESCENTES

(30) Priorität: 12.09.2005 AT 14882005
(43) Veröffentlichungstag der Anmeldung: 04.06.2008
(73) Patentinhaber: Universität Innsbruck, 6020 Innsbruck (AT)
(72) Erfinder: KRÄUTLER, Bernhard, A-6020 Innsbruck (AT); MÜLLER, Thomas, A-6020 Innsbruck (AT)
(74) Vertreter: Schwarz & Partner
(86) Internationale Anmeldenummer: PCT/AT2006/000370
(87) Internationale Veröffentlichungsnummer: WO 2007/030848

(56) Entgegenhaltungen:
- US-A1- 2003 023 081
- C. CURTY; N. ENGEL: "Detection, Isolation and Structure Elucidation of a Chlorophyll a Catabolite from Autumnal Senescent Leaves of Cercidiphyllum Japonicum" PHYTOCHEMISTRY, 1996, Seiten 1531-1536, XP002420696 in der Anmeldung erwähnt
- B.KRÄUTLER: "Unravelling Chlorophyll Catabolism in Higher Plants" BIOCHEMICHAL SOCIETY TRANSACTIONS, 2002, Seiten 625-630, XP002420697
- M. OBERHUBER, J. BERGHOLD, W. MÜHLECKER, S. HÖRTENSTEINER, B. KRÄUTLER: "Chlorophyll Breakdown - on a Nonfluorescent Chlorophyll Catabolite from Spinach" HELVETICA CHIMICA ACTA, 2001, Seiten 2615-2627, XP002420698

## Beschreibung

Die Erfindung betrifft nichtfluoreszierende Chlorophyllkataboliten (NCC), deren Verwendung sowie ein Verfahren zur Gewinnung derartiger Chlorophyllkataboliten.

Der Chlorophyllabbau ist ein jahreszeitlich bedingtes Ereignis, bei dem jährlich auf der gesamten Erde ca. 10⁹ Tonnen Chlorophyll abgebaut werden. Was mit dem Chlorophyll tatsächlich passiert, wenn im Herbst die Blätter gelb werden, war lange Zeit unbekannt. Erst in den letzten beiden Jahrzehnten ist es gelungen, die Spur des Chlorophylls nach dem Verschwinden der grünen Farbe weiterzuverfolgen [Kräutler, Bernhard. Chlorophyll Breakdown and Chlorophyll Catabolites. In: The Porphyrin Handbook. Kadish, K.M., Smith, K.M., Guilard R. (Hrsg), Elsevier Science 2003, Vol. 13, Kapitel 82, S. 183-209.]. Vergleicht man diesen komplexen Vorgang mit einem Puzzle, so ist es gelungen, viele wichtige aber noch nicht alle Teile dieses Puzzles zusammenzusetzen. Am Ende dieser Abbaukette stehen nach heutigem Erkenntnisstand die so genannten "Nichtfluoreszierenden" Chlorophyllkataboliten, kurz NCCs, die man in den Vakuolen von gealterten Pflanzenzellen finden kann und von denen man bis dato etwa zwölf strukturell leicht unterschiedliche NCCs kennt, die aus den Blättern verschiedener seneszenter Pflanzen isoliert werden konnten. Funktionell ist allerdings nur wenig über diesen Verbindungstyp bekannt.

Die Erfindung beruht auf der überraschenden Erkenntnis, dass derartige NCCs in reifen Früchten z.B. in Äpfeln und Birnen enthalten sind, insbesondere in den Schalen von derartigen Früchten, und deshalb auch verzehrt werden. Die Erkenntnis, dass NCCs einen Bestandteil der menschlichen Nahrungskette bilden, ist ein Hinweis, dass NCCs einerseits keine toxischen Eigenschaften aufweisen und andererseits einen wichtigen Bestandteil der menschlichen Nahrung mit noch unbekannter Wirkung darstellen.

Aufgrund dieser Erkenntnis, die der Bedeutung dieser Verbindungen eine völlig neue Dimension verschafft, stellte sich die Frage, ob diesem bis dato nicht beachteten Naturstoff eine bestimmte Rolle in unserem Stoffwechsel zukommt?

Die Extraktion und im Speziellen die Reinigung dieser Verbindungen gestaltet sich in den meisten Fällen sehr aufwendig. Die seneszierenden Blätter eines Baumes namens Cerciphyllum Japonicum stellen eine günstige Quelle zur Isolation von NCCs dar. Das Chlorophyll wird im Falles dieses Baumes, der im Deutschen Judasblattbaum oder Lebkuchenbaum genannt wird, nämlich nur zur zwei unterschiedlichen Verbindungen abgebaut, größtenteils zu Cj-NCC-1 (im folgenden Text immer als NCC-1 bezeichnet) [Oberhuber, M., Berghold, J., Breuker, K., Hörtensteiner, S., Kräutler, B. Breakdown of Chlorophyll: A Nonenzymatic Reaction Accounts For the Formation of The Colorless "Nonfluorescent" Chlorophyll Catabolites. Proc. Natl. Acad. Science U.S.A. (2003), Vol. 100, S. 6910-6915.]. Die erstmalige Isolierung und Charakterisierung von Cj-NCC-1 wurde von Curty und Engel im Jahre 1996 beschrieben [Curty, C. und N. Engel. Detection, Isolation and Structure Elucidation of a Chlorophyll a Catabolite from Autumnal Senescent Leaves of Cercidiphyllum Japonicum, Phytochemistry (1996), Vol 42, No. 6, S. 1531-1536.].

Alle bisher publizierten Verfahren zur Isolierung von NCCs aus Pflanzenmaterialien sind nur dazu geeignet, diese Verbindungen im Milligramm-Maßstab (im Bereich von 1-20mg NCC) für die Strukturanalyse (z. B. NMR, UV) herzustellen. Die Arbeitsschritte sind zeit- und arbeitsaufwenig und für die Isolierung größerer Mengen an reinen NCCs ungeeignet. Das ist vor allem darauf zurückzuführen, dass bei allen diesen Verfahren entweder die präparative Dünnschichtchromatographie (TLC) bzw. die präparative HPLC zum Einsatz kommen und beide Verfahren ein Upscaling unmöglich machen. Daneben ist bei der Isolierung von NCCs mittels präparativer TLC erfahrungsgemäß eine oxidative Zersetzung zu farbigen Abbauprodukten nicht zu verhindern. Da die Strukturaufklärung von neuen Chlorophyllkataboliten bis dato im Mittelpunkt des wissenschaftlichen Interesses stand, war die Herstellung bzw. Isolierung von größeren Mengen nicht gefragt.

Aufgabe der Erfindung ist daher die Entwicklung eines neuen Verfahrens, mit dem größere Mengen an NCCs auf einfache Art und Weise gewonnen werden können mit einem deutlich höheren Reinheitsgrad als beim Stand der Technik.

Das erfindungsgemäße Verfahren zur Gewinnung von nichtfluoreszierenden Chlorophyllkataboliten mit der chemischen Formel I 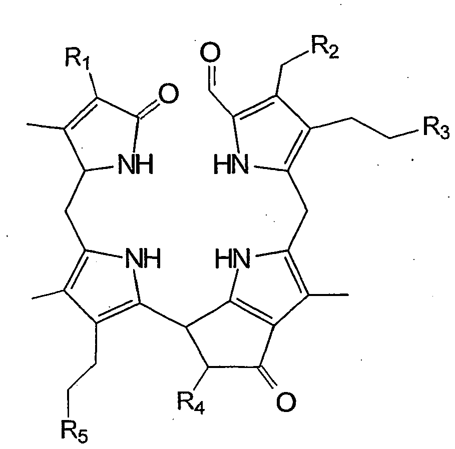 oder ein pharmakologisch, dermatologisch oder kosmetisch verträgliches Salz oder Derivat davon, wobei R₁ bis R₅ ausgewählt ist aus den Resten
- R₁: -Alkyl, -Vinyl, -CHOH-CH₂OH
- R₂: -H, -OH, -OAlkyl, -OAcyl, Saccharidreste, modifizierte Saccharidreste (z.B. malonyliert)
- R₃: -H, -OH, -OAlkyl, -OAcyl, modifizierte -OAcyl (z.B. malonyliert), Saccharidreste, modifizierte Saccharidreste (z.B. malonyliert)
- R₄, R₅: -COOH, -Carbonsäureester
sowie dessen Salze und Solvate aus pflanzlichem Material, insbesondere aus Pflanzenblättern zeichnet sich aus durch die Schritte:
- Extraktion des pflanzlichen Materials mit einem ersten Lösungsmittel oder Lösungsmittelgemisch mit einer Dielektrizitätskonstante von 5 bis 20 und einem Dipolmoment von 5 bis 10 × 10⁻³⁰ Cm zu einem Rohextrakt
- Filtration oder Chromatographie dieses Extraktes über Kieselgel
- Elution des Kieselgels mit einem zweiten Lösungsmittel oder Lösungsmittelgemisch, vorzugsweise mit größerem Dipolmoment als das erste Lösungsmittel
- Abziehen des Lösungsmittels und gegebenenfalls Kristallisation des erhaltenen Rückstandes.

Mit einem solchen Verfahren ist es möglich, größere Mengen an NCCs der Formel I herzustellen.

Insbesondere hat es sich als günstig erwiesen, wenn als erstes Lösungsmittel CH₂Cl₂, Methylacetat, Ethylacetat, t-Butanol, Ethylmethylketon oder Mischungen daraus verwendet wird. Methylenchlorid eignet sich dabei besonders gut, allerdings hat es sich auch herausgestellt, dass Methylacetat und Ethylacetat ebenfalls sehr gut geeignete Lösungsmittel sind, wobei es sich bei diesen beiden Lösungsmitteln vorteilhafterweise um nicht halogenierte Lösungsmittel handelt, was spätere Verwendungsmöglichkeiten der aus einem solchen Verfahren gewonnenen Verbindung verbessert.

Aus der Extraktion des pflanzlichen Materials mit dem ersten Lösungsmittel ergibt sich eine Lösung, die auch sehr viele unerwünschte Komponenten beinhaltet. Um Substanzen, die auch gut in Wasser löslich sind, abzutrennen, hat es sich daher als vorteilhaft erwiesen, wenn vor dem Filtrationsschritt ein Reinigungsschritt des Rohextraktes mit einem nicht mischbaren Lösungsmittel erfolgt.

Für das erfindungsgemäße Verfahren ist außerdem vorgesehen, dass der Filtrationsschritt mit einer kieselgelgefüllten Chromatographiesäule durchgeführt wird. Ein solcher Filtrationsschritt über eine Chromatographiesäule hat den Vorteil, dass die Bedingungen sehr konstant gehalten werden können (gleichmäßiger Durchfluss, gleichmäßig gepackte Säule) und man Standardgeräte verwenden kann.

Bei dem Filtrationsschritt über Kieselgel hat es sich bei direkter Aufgabe des gelösten Extraktes gezeigt, dass nichtfluoreszierende Chlorophyllkataboliten gut daran haften, während zahlreiche andere Verbindungen, die im ersten Extrakt gelöst sind, rascher von der Säule eluieren. Der Elutionsschritt des Kieselgels, d.h. das eigentliche Herunterspülen des NCC von Kieselgel, erfolgt daher günstigerweise mit einem Lösungsmittel, das diese Verbindungsklasse wieder vom Kieselgel löst. Günstig hat es sich dabei erwiesen, wenn als zweites Lösungsmittel ein Lösungsmittel größerer Polarität als das erste Lösungsmittel verwendet wird und aus der Gruppe Aceton, Ethylmethylketon, Methanol, Methanol-Wasser-Gemische, Methylacetat oder Mischungen davon oder aus Mischungen des ersten Lösungsmittels oder Lösungsmittelgemisches mit einem Lösungsmittel größerer Polarität stammt.

Auch nach dem Eluationsschritt des Kieselgels befinden sich immer noch einige Substanzen im Eluat, die mit einer wässrigen Lösung entfernt werden können.

Nachdem das Lösungsmittel im letzten Schritt verdampft ist, beispielsweise durch einen Rotationsverdampfer, hat es sich günstig erwiesen, wenn der Rückstand aus dem Verdampfungsschritt kristallisiert wird und anschließend filtriert und getrocknet wird. Dies erhöht deutlich die Haltbarkeit von NCCs extrem, da sich verunreinigte NCCs an der Luft sehr rasch zersetzen und verfärben.

Für den Filtrationsschritt hat es sich außerdem als günstig erwiesen, wenn das Kieselgel eine Korngröße von unter 5000, vorzugsweise von unter 1000, aufweist.

Weiters hat sich als vorteilhaft erwiesen, dass als pflanzliches Material Pflanzenteile verwendet werden, die vor der Seneszenz (Reifung) grün sind, da in den grünen Pflanzenbestandteilen naturgemäß die Chlorophyllmenge besonders groß ist. Insbesondere ist es günstig wenn als pflanzliches Material im Wesentlichen senesziertes oder gereiftes Pflanzenmaterial verwendet wird, da dann das Chlorophyll zumindest bereits teilweise zu NCC katabolsiert wurde. Da sich NCCs in geernteten Pflanzen rasch zersetzen, ist es vorteilhaft, wenn als Pflanzenmaterial frisch geerntetes, senesziertes oder gereiftes Pflanzenmaterial oder senesziertes oder gereiftes Pflanzenmaterial verwendet wird, das nach der Ernte bei etwa -20°C oder darunter gelagert wurde. Es hat sich herausgestellt, dass besonders große Mengen an NCCs dann gewonnen werden konnten, wenn das Pflanzenmaterial aus Agrarpflanzen wie Ölraps, Klee, Gerste, Mais, Tabak, von krautartigen Pflanzen und landwirtschaftlich produzierten Gemüsepflanzen wie Spinat, Brokkoli, Paprika, von Sträuchern und Laubbäumen wie Hamamelis, Ulmen, Platanen, Nussbäumn, *Cercidiphyllum japonicum* und von Nadelbäumen wie Lärchen stammt.

Das neue Verfahren zeichnet sich durch einfache Methoden und automatisierbare Arbeitsschritte aus, was erst die Reinisolation und Kristallisation der chemisch labilen (oxidationsempfindlichen) Verbindungen erlaubt. Upscalingversuche mit größeren Arbeits- und Glasgeräten haben gezeigt, dass das Verfahren bei Verwendung von recyclierbaren Lösungsmitteln (Kosteneffizienz) sehr gut und praktisch verlustfrei dazu geeignet ist, große Mengen an NCCs mit hoher Reinheit im Gramm-Maßstab (und größeren Mengen) herzustellen.

Das hier beschriebene Verfahren erlaubt eine rasche, einfache und leicht upscalebare Isolierung von NCCs aus gewöhnlichen Pflanzenmaterialien in hoher Reinheit. Derzeit ist noch keine Methode bekannt, die eine Reinisolation von NCCs und Kristallisation von NCCs in großen Mengen erlaubt.
- Aufgrund der schnellen Verarbeitung und der Vorgangsweise bei entscheidenden Arbeitsschritten kann erstmals die störende Bildung von strukturell verwandten, farbigen Zersetzungsprodukten völlig unterdrückt werden.
- Die Kristallisation als letzter Arbeitsschritt gewährt höchste Reinheit der isolierten Verbindungen.
- Halogenierte Kohlenwasserstoffe (wie Dichlormethan oder Chloroform) sind gleichzeitig Lösungsmittel für die Extraktion und Chromatographie, sie sind leicht recyclierbar.
- Halogenierte Lösungsmittel sind aber auch durch chlorfreies Ethylacetat bzw. Methylacetat ersetzbar.
- Seneszente Blätter (z.B. die des Baumes Cercidiphyllum japonicum) sind eine sehr billige, und (fast) unerschöpfliche Quelle für NCCs (z.B. NCC-1). Dieselbe Sorte von NCCs wurde von uns vor kurzem auch in Früchten gefunden. Die Früchte selbst eignen sich aber nicht für eine Isolation größerer Mengen an NCCs, weil diese dort nur in sehr kleinen Mengen nachgewiesen werden konnnten.

Beispiel für die Präparierung von NCC-1 aus seneszenten Blättern von Cercidiphyllum japonicum:

1 kg kaltes Blattmaterial (Blätter vom Botanischen Garten der Universität Innsbruck, Österreich, gesammelt am 23.10.03, Farbe: gelb-grün, gelagert im Tiefkühler bei -80°C) wurde in zwei Ansätzen zu je 0,5 kg in einem 5 L Behälter aus Stahl mit 1,4 L kaltem (5°C) Methylenchlorid und 25 g Ascorbinsäure 5 min fein gemixt (Braun Vario 600W) und über einen 1cm Cellitefilter (Durchmesser 14cm) filtriert. Der Filterkuchen wurde noch zweimal mit je 0,7 L kaltem Methylenchlorid gemixt, neuerlich filtriert und mit 100 mL kaltem Methylenchlorid nachgewaschen.

Die erhaltene klare Lösung (dunkelgrün) wurde nun in einen Scheidetrichter transferiert und mit 1,5 L Kaliumphosphatpuffer (c= 100 mM, pH= 5,2; auf 1 L Puffer wurde 1 g Ascorbinsäure zugegeben) extrahiert. Die organische Phase wurde über getrocknete Watte (2 Tage im Trockenschrank bei 100°C) in einen 3 L Erlenmeyerkolben abgelassen. Die wässrige Phase wurde mit 200 mL und 100 mL Methylenchlorid zurückextrahiert und die Methylenchloridphase ebenfalls über getrocknete Watte abfiltriert. Die Watte wurde danach noch mit 100 mL Methylenchlorid nachgewaschen. Die beiden so erhaltenen Extrakte (von jeweils 0,5 kg Ausgangsmaterial) wurden vereinigt, gekühlt und auf die vorbereitete Chromatographiesäule aufgetragen.

Säule für die Chromatographie wurde wie folgt vorbereitet:
Säulenhöhe: <=60 cm; Säulendurchmesser: >=7 cm
Kieselgelschicht: <=38 cm; Seesandschicht: 0,5cm
Laufmittel für die Säulenpackung: 100 % Methylenchlorid dest.

Die leere Chromatographiesäule wurde mit 500 mL Laufmittel beschickt und die Luft mittels Handpumpe unter der Fritte entfernt. 300-700 g Kieselgel 60 wurden mit 1,5 L Methylenchlorid dest. im Ultraschallbad entgast und in die Säule eingefüllt. Das Laufmittel wurde unter ständigem Klopfen bis 3cm über der Kieselgelschicht abgelassen. Anschließend wurden 0,5 cm Seesand aufgegeben und die Säule im Kühlraum gekühlt. Die Chromatographie wurde bei etwa 4 Grad im Kühlraum durchgeführt.

Verwendete Laufmittel:
Laufmittelgemisch **1** : 95 % Methylenchlorid dest., 5 % Methanol dest. (insgesamt 5 l)
Laufmittelgemisch **2** : 90 % Methylenchlorid dest., 10 % Methanol dest. (insges. 2,5 l)

Das Aufgeben des Extraktes auf die Säule benötigte ca. 1-3h. Es wurde dann mit 100 mL reinem Methylenchlorid dest., gewaschen, sodass der Überstand über dem Sand farblos war. Nun wurde die Säule mit Laufmittelgemisch **1** aufgefüllt (Gemisch aus 95% Methylenchlorid dest., 5% Methanol dest.). Nachdem das Chlorophyll eluiert worden war (dunkelgrüne Fraktionen), wurden Fraktionen von jeweils ca. 200 mL entnommen und mittels DC analysiert (Polygramm SIL G/UV 254 Schichtdicke 0,2 mm Kieselgel mit Fluoresentindikator, Lösungsmittel: Chloroform : Methanol : Wasser = 95 : 10 : 1; mit der Referenzsubstanz Cj-NCC-1). Nach der ersten reinen NCC-1 Fraktion (DC Kontrolle), wurde das Laufmittel auf Laufmittelgemisch **2** (90% Methylenchlorid dest., 10% Methanol dest.) geändert. Es wurden Fraktionen gesammelt, bis kein NCC-1 mehr von der Säule herunterkam (DC Kontrolle). Jene Fraktionen (insgesamt 2150 mL), die laut DC reinen NCC-1 enthielten, wurden in einem Scheidetrichter vereinigt und mit 1 L Kaliumphosphatpuffer (c= 100 mM, pH= 5,2 /+1g Ascorbinsäure auf 1 L Puffer) extrahiert. Die org. Phase wurde in einen 3 lit. Erlenmeyerkolben abgelassen und die wässrige Phase mit 200 mL Methylenchlorid dest. zurückextrahiert. Die org. Phasen wurden im 3 L Erlenmeyerkolben vereinigt und über getrocknete Watte in zwei 2 L Rundkolben filtriert. Die Watte wurde mit ca. 100 mL Methylenchlorid dest. nachgewaschen. Nun wurde das hellgelbe Filtrat am Rotationsverdampfer (25°C Badtemperatur, p= 400mbar, unter Lichtschutz!) bis auf ca. 100 mL eingeengt, in einen 250 mL Rundkolben überführt und erneut am Rotationsverdampfer bis zur Trockene eingedampft.

Der Rückstand wurde in 5 mL Methylenchlorid dest. (über Aluminiumoxid basisch filtriert) gelöst und mit n-Hexan (purum) im 250 mL Rundkolben bis zum Rand aufgefüllt, der NCC-1 fiel dabei als feiner gelblich-weißer Niederschlag aus.

### Reinisolation und Charakterisierung

Der Inhalt des Rundkolbens wurde über einen Papierrundfilter (5 cm) abfiltriert und der Rückstand in ein Pillenglas überführt. Das Produkt wurde am Rotationsverdampfer 15 min. vorgetrocknet und danach für 3 h am Hochvakuum getrocknet.

Ausbeute: 491 mg NCC-1 (als feinkristallines Pulver)

Das erhaltene Produkt wurde mittels DC und UV charakterisiert.

Das erhaltene Produkt lässt sich in einem Reinheitsgrad von über 95%, bevorzugt über 99%, besonders bevorzugt über 99,5% (Gewichtsprozente), gewinnen. NCC-1 ist dadurch gekennzeichnet, dass R₁ = -Vinyl, R₂ = -H, R₃ = OH, R₄ = -COOCH₃, und R₅ = -COOH entspricht.

Überraschend war die Erkenntnis, dass in Äpfeln (Malus sylvestris) bzw. Birnen (Pyrus communis) ein NCC gefunden wird, der identisch mit dem verhältnismäßig leicht zugänglichen NCC-1 aus dem Judasblattbaum ist. Damit ist auch für uns der praktische Weg geöffnet, um mit diversen Experimenten die chemischen Eigenschaften bzw. biologische Bedeutung eines bislang unbeachteten Nahrungsbestandteiles zu prüfen. Ein "Golden Delicious" Apfel (mit einem Durchmesser von ca. 8-10cm) kann aufgrund der vorhandenen Chlorophyllmenge (laut Zude-Sasse, M., Truppel, I. and B. Herold. An approach to nondestructive apple fruit chlorophyll determination. Postharvest Biology and Technology 25 (2002), 123-133) höchstens ca. 0,1 - 1mg NCCs enthalten.

Weitere Details und Vorteile der Erfindungen sowie Ausführungsbeispiele werden anhand der Figuren und Figurenbeschreibungen unter folgenden Ausführungen näher erläutert.

Es zeigt die
- Fig. 1: Untersuchungen zur Quantifizierung der antioxidativen Wirkung von Bilirubin bzw. NCC-1,

- Fig. 2: den Effekt von NCC-1 auf das Wachstum von Brustkrebszellen der Linie MDAMB231,
- Fig. 3A bis C: die Induktion von Zelltod in verschiedenen Krebszelltypen, analysiert bei Flusszytometrie,
- Fig. 4: den Effekt von NCC-1 auf die Apoptose in CCRF-CEM-Zellen analysiert m mittels Flusszytometrie,
- Fig. 5A bis 5F: den Effekt von NCC-1-Behandlung auf den Zellzyklus in CCRF-CEM-Zellen, und
- Fig. 6: den Effekt von NCC-1 auf die Proteinexpression von p27/KIP in CCRF-CEM-Zellen (Western-Blot).

Es ist seit einiger Zeit bekannt (z.B. bei der Dünnschichtchromatographie), dass sich auf Kieselgel NCCs rasch an der Luft (und am Tageslicht) in rostfarbene Produkte zersetzen, wobei eine Oxidation als Merkmal für die Zersetzung vermutet wird. Nach den bisher bekannten Reinigungsverfahren konnten immer nur NCCs gewonnen werden, die durch Zersetzungsprodukte verunreinigt waren und sich bei der Lagerung innerhalb mehrerer Stunden zersetzten. Aufgrund dieser Tatsache war bekannt bzw. davon auszugehen, dass NCC-Rohisolate unter Standardbedingungen (d.h. bei Raumtemperatur und in der Anwesenheit von Luftsauerstoff) zu instabil sind, als dass ihnen eine gezielte Anwendung zukommen würde. In einer ersten überraschenden Erkenntnis konnte nun gezeigt werden, dass NCC unter physiologischen Bedingungen (in Lösung, organische bzw. biologische Matrix; siehe unten) eine antioxidative Wirkung besitzen. Aufgrund dieser überraschenden Erkenntnis wurden intensivere Untersuchungen zur antioxidativen Wirkung von NCCs durchgeführt. Auch die Erkenntnis, dass kristallisierte NCCs in gereinigter Form, wie sie im erfindungsgemäßen Verfahren gewonnen werden, in gekühltem Zustand gut haltbar sind (praktisch unzersetzt aufbewahrt werden können) trotz der Anwesenheit von Sauerstoff, ermöglicht erst eine gezielte Anwendung von Repräsentanten dieser Verbindungsklasse.

Da eine gewisse chemische und biologische Verwandtschaft zwischen Hämkataboliten (wie Bilirubin) und Chlorophyllkataboliten (den NCCs) besteht, war es besonders interessant, die NCCs auf ihre antioxidative Wirksamkeit zu überprüfen, was in Anlehnung an bereits etablierte Experimente von Stocker et al. über die Wirkung von Bilirubin nachzuweisen war [Stocker, R., Yamamoto, Y., McDonagh, A.F., Glazer, A., Ames, B.N. Bilirubin is an Antioxidant of Possible Physiological Importance. Science (1987), Vol. 235, S. 1043-1046.]. Sowohl Chlorophyll als auch Häm gehören zur Klasse der so genannten porphyrinoiden Naturstoffe. Häm ist ein tetrapyrrolisches Ringsystem, das als Hämoglobin mit Hilfe eines komplexierten Eisenatoms in tierischen Organismen Sauerstoff transportieren kann. Zentrale Strukturelemente eines Chlorophyllmoleküls sind ebenfalls ein tetrapyrrolisches Ringsystem und ein komplexiertes Magnesiumatom, das in pflanzlichen Chloroplasten kontrolliert Lichtenergie aufzunehmen vermag. Sowohl pflanzliche als auch tierische Organismen haben im Laufe der Evolution Wege gefunden, um diese Naturstoffe unter bestimmten Bedingungen gezielt und kontrolliert zu linearen Tetrapyrrolen abzubauen bzw. auszuscheiden.

Während die Abbauprodukte des Chlorophylls als Nichtfluoreszierende Chlorophyllkataboliten (NCCs) in der pflanzlichen Vakuole gelagert werden, kann das abgebaute Häm in Form von einem Bilirubin-Konjugat über die Nieren ausgeschieden werden. Im Falle tierischer Organismen, war die Bedeutung der so genannten Biliverdin-Reduktase, einem Enzym, welches das Biliverdin zu dem in größeren Konzentrationen toxisch wirkenden Bilirubin reduziert, seit längerer Zeit ein Rätsel. 2002 konnte jedoch gezeigt werden, dass der Redoxzyklus Biliverdin-Bilirubin eine wichtige Rolle bei dem Schutz von Zellen gegenüber oxidativem Stress spielt [Baranano, D.E., Rao, M., Ferris, C.D., Snyder, S.H. Biliverdin Reductase: A Major Physiologic Cycloprotectant. PNAS (2002), Vol. 99, No. 25, S. 16093-16098.]. Wesentlich dabei ist die antioxidative Wirkung von Bilirubin, die bereits seit 1987 bekannt ist [Stocker, R., Yamamoto, Y., McDonagh, A.F., Glazer, A., Ames, B.N. Bilirubin Is an Antioxidant of Possible Physiological Importance. Science (1987), Vol. 235, S. 1043-1046.].

Stocker et al. verwendeten zur Quantifizierung der antioxidativen Wirkung des Hämabbauproduktes Bilirubin ein einfaches Testsystem. Die essentielle Fettsäure Linolsäure, eine zweifach ungesättigte Fettsäure, ist sehr empfindlich gegenüber einer Oxidation durch freie Radikale. Derartige Oxidationen können in Form von Kettenreaktionen z.B. in Zellmembranen großflächige Schäden herbeiführen. In vitro wurde dieser "oxidative Stress" durch den Einsatz einer Azoverbindung (Radikalstarter) simuliert. Derartige Azoverbindungen bilden ständig, abhängig von der Temperatur durch Abspaltung von Stickstoff (N₂) Kohlenstoffradikale, die wiederum mit den ungesättigten Bindungen der Fettsäuren reagieren können. Es bilden sich Fettsäureradikale die ihrerseits wiederum mit Sauerstoff reagieren. Das führt zur Bildung von Fettsäurehydroperoxiden, die wegen ihrer besonderen Absorptionseigenschaften in UV-Bereich (234 nm) spektroskopisch nachweisbar sind. Abhängig von der Konzentration des Radikalbildners steigt also mit der Zeit die Anzahl der Fettsäurehydroperoxidmoleküle an. Setzt man einen antioxidativ wirksamen Stoff zu, können Radikale abgefangen werden, und die Bildung der Hydroperoxide wird verlangsamt.

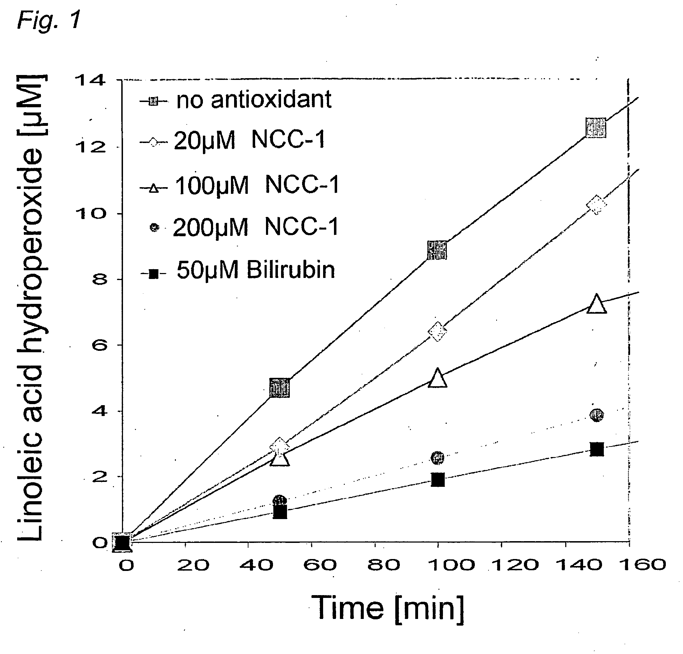

Das Ergebnis dieser Untersuchungen zeigt die Figur 1, in der die zeitliche Abhängigkeit der Zunahme oxidierter Linolsäure bei unterschiedlichen Konzentrationen der Antioxidantien Bilirubin bzw. NCC-1' aufgetragen ist.

Unsere Versuche haben eindeutig gezeigt (vgl. Fig. 1; aufgetragen ist die Konzentration an Linolsäure in µM gegen die Zeit in Minuten, gezeigt sind die Funktionen ohne Antioxidans, mit 20, 100 und 200 µM NCC-1 sowie 50 µM Bilirubin), dass der Chlorophyllkatabolit NCC-1 ebenso wie Bilirubin als Antioxidans wirkt. Wie der Fig. 1 zu entnehmen ist, ist ein 200µM Zusatz von NCC-1 mit einem 50µM Zusatz von Bilirubin zu vergleichen. Ohne diesen Zusatz von 200µM NCC-1 bzw. 50µM Bilirubin ist die Oxidation von Linolsäure etwa viermal schneller (oberste Kurve).

Detaillierte experimentelle Beschreibung der antioxidierenden Wirkung von NCCs-1:

Die Wirkung als Antioxidans des nicht fluoreszierenden Chlorophyllkataboliten NCC-1 wurde mittels der Autoxidationsreaktion von Linolsäure untersucht, als Radikalbildner wurde Azobisisobutyronitril verwendet. NCC-1 inhibiert die Oxidation dieser Fettsäure (bei einer Konzentration von 200 µm und bei Raumtemperatur ungefähr um den Faktor 4). Die antioxidative Wirkung von NCC-1 ist in diesen standardisierten Modellen ungefähr vergleichbar mit der des natürlich vorkommenden Antioxidans Bilirubin.

Materialien: Die Lösungsmittel für die Extraktionsschritte waren vom Einheitsgrad "reagentgrade" und wurden vor der Benutzung destilliert. Methanol stammte von Merck Darmstadt, Deutschland und Acros Organics, Geel, Belgien und war von HPLC-Qualität. 99 %-iges Bilirubin und 99 %-ige Linolsäure wurden ebenfalls von Acros Organics bezogen, Aluminiumoxid (basisch) für Chromatographie, Chlorophorm puriss. p.a., Kaliumdihydrogenphosphat puriss. p.a. und Kaliumphosphat dibasisch wasserfrei puriss. p.a. stammten von Fluka, Buchs, Schweiz. Sep-Pak-C18 Cartridges stammten von Waters Asociates. Die pH-Messungen wurden mit einer WTW Sentix 21 Elektrode angeschlossen an ein WTW pH535 digital pH Meter durchgeführt.

HPLC: Gynotek HPLC System mit manueller Probeentnahmevorrichtung, M480 Pumpe (analytisch), M300 Pumpe (präparativ), Phenomenex DG-301 online Entgaser, UVD 340 Diodenanordnungsdetektor und Jasco FP-920 Fluoreszenzdetektor. Die Daten wurden mittels Gynkosoft 5.50 gesammelt und mit Chromelion V6.50 verarbeitet. HP 1100 System mit manueller Probenentnahme, online Entgaser und Diodenanordnungsdetektor. Die Daten wurden gesammelt und verarbeitet mit HP Chemstation for 3D. LC Packings Ultimate Nano-HPLC-System mit Dionex UVD 340S Diodenanordnungsdetektor für die LC-MS Experimente. Die Daten wurden gesammelt und verarbeitet mit Chromeleon V6.50. Analytische HPLC: Hypersil ODS 5µm 250 x 4,6 mm im Durchmesser Säule bei Raumtemperatur geschützt durch eine Phenomenex ODS 4 mm x 3 mm im Durchmesser Vorsäule, Flussrate 0,5 mm pro Minute, 20 µl Injektionsvolumen. Lösungsmittel A: 100mM Kaliumphosphatpuffer (pH 7,0), Lösungsmittel B: Methanol. Präparative HPLC: Hypersil ODS 5 µm 250 mm x 21,2 mm im Durchmesser Säule bei Raumtemperatur, Flussrate 5 mm pro Minute, die Lösungsmittel wurden durch ein Ultraschallbad entgast. Nano-HPLC: Nucleosil 125-5 50 mm x 100 µm im Durchmesser Kapillarensäule bei Raumtemperatur, Flussrate 300 µl pro Minute, 1 µl Injektionsvolumen. Lösungsmittel A: Wasser, Lösungsmittel B: Methanol.

UV/Vis: Hitachi U-3000 Spektrophotometer; λₘₐₓ(nm)/(rel.ε).

NMR: Varian Unityplus 500 MHz; δ(H) in ppm referenziert auf δ (C¹HD₂OD)=3,31 ppm, Kopplungskonstanten J in Hz, die Spektren wurden bei 26°C aufgezeichnet.
MS: Finigan MAT 95-S in Positiv-Ionen Modus; FAB-MS mit Cäsiumquelle; 20 keV, Glycerin als Matrix; ESI-MS: Picoview Nanosource, Flussrate 300 µl pro Minute, Sprühspannung 1,2 kV, Lösungsmittel Wasser/Methanol 1:2 (v/v).

Bestimmung der Wirkung von NCC-1 als Antioxidans (nach R. Stocker, Y. Yamamoto, A. F. McDonagh, A. N. Glazer, B.N. Ames, Science 235, 1043 (1987)). Stocklösungen von Linolsäure, Azobisisobutyronitril (AIBN), NCC-1 und Bilirubin wurden hergestellt, indem 470 µl Linolsäure in 1530 µl Chloroform, 6,9 mg AIBN in 2030 µl Chloroform, 0,5 mg NCC-1 in 300 µl Chloroform und 0,4 mg Bilirubin in 550 µl Chloroform gelöst wurden. Die genauen Konzentrationen von NCC-1 und Bilirubin in den Stocklösungen wurden mittels UV/Vis Spektroskopie bestimmt (50 µl der Stocklösung wurden mit 3 mL Methanol verdünnt; ε (NCC) in Methanol bei 310 nm beträgt 15000; ε (Bilirubin) in Methanol bei 450 nm beträgt 55000). Die Reaktionslösungen wurden hergestellt, indem Aliquote der Stocklösungen zu den folgenden Konzentrationen gemischt wurden: 0,15 M Linolsäure, 2 mM AIBN und 0 - 200 µM NCC-1 bzw. Bilirubin. Die Reaktionslösungen wurden bei 37°C gehalten, die Bildung von Linolsäure Hydroperoxid wurde mittels UV/Vis Spektroskopie bzw. HPLC Analyse bestimmt.

UV/Vis Spektroskopie: 40 µl der Reaktionsmischung wurden mit 3 mL Chloroform verdünnt. Die Absorption wurde im Wellenlängenbereich 200 - 800 nm gegen Methanol gemessen, die Gesamtabsorption bei 234 nm wurde zur Bestimmung des Reaktionsverlaufes herangezogen.

Analytische HPLC wurde auf einer 250 mm x 4,6 mm im Durchmesser Hypersil ODS 5 µm Säule bei Raumtemperatur durchgeführt.
Das Eluierungsmittel für die isokratische Trennung war Methanol mit einer Flussrate von 1 mL pro Minute. 40 µl der Reaktionsmischung wurden mit 1,5 mL Chloroform verdünnt, 20 µl dieser Lösung wurden auf die HPLC aufgetragen.

Weitere Vorteile der Erfindung sind im Folgenden dargestellt:
- Die NCCs sind farblose und geruchlose Naturstoffe, sie sind in Spuren Bestandteil unserer Nahrung.
- Die NCCs haben antioxidative Wirkung vergleichbar mit der von Bilirubin. Im Gegensatz zu Bilirubin (Gelbsucht!) sind über NCCs keine schädlichen Eigenschaften bekannt.
- Pflanzen bieten eine schier unerschöpfliche Quelle für diese Substanzen, die Extraktion bzw. Reinigung ist bei der Wahl des richtigen Pflanzentyps verhältnismäßig einfach.
- Chlorophyllkataboliten decken ein sehr großes Löslichkeitsspektrum ab, sie könnten also praktisch universell eingesetzt werden, d.h. sowohl in wässrigen also polaren Systemen, wie auch in fettähnlichen also apolaren Lösungsmitteln.
- Chemische Modifikationen sind an NCCs möglich, dadurch erweitert sich natürlich auch das Spektrum der Löslichkeits- und Polaritätseigenschaften.
- Sie könnten überall dort zum Einsatz kommen wo oxidationsempfindliche Naturprodukte und Derivate davon länger haltbar gemacht werden sollten.

Bei der Entwicklung des neuen Verfahrens wurde vor allem auf folgende Punkte Wert gelegt:
(i) Wahl einfacher und leicht upscalebarer Methoden (vor allem keine präparative TLC und keine präparative HPLC).
(ii) Schnelle Isolation und Aufarbeitung im Hinblick auf die geringere Empfindlichkeit der Substanz in gereinigter Form (im Pflanzenextrakt enthaltene Enzyme bzw. Fremdsubstanzen aus den Pflanzen können die Zerstörung und Oxidation der NCCs beschleunigen).
(iii) Unterdrückung der Bildung von farbigen Zersetzungsprodukten während der Aufarbeitung (z.B. durch Kühlung während bestimmter Arbeitsschritte).
(iv) Feinkristallisation als letzter Reinigungsschritt und damit die Gewährleistung höchster Reinheit und verbesserter Lagerfähigkeit der an sich labilen, oxidationsempfindlichen Substanz.

Aufgrund der Eigenschaften von NCCs als Antioxidans ist ein weiterer Aspekt der Erfindung eine Zubereitung, enthaltend zumindest eine Verbindung der Formel I 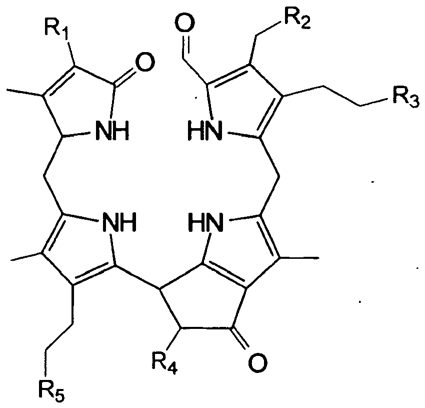 oder ein pharmakologisch, dermatologisch oder kosmetisch verträgliches Salz oder Derivat davon, wobei R₁ bis R₅ ausgewählt ist aus den Resten
- R₁: -Alkyl, -Vinyl, -CHOH-CH₂OH
- R₂: -H, -OH, -OAlkyl, -OAcyl, Saccharidreste, modifizierte Saccharidreste (z.B. malonyliert)
- R₃: -H, -OH, -OAlkyl, -OAcyl, modifizierte -OAcyl (z.B. malonyliert), Saccharidreste, modifizierte Saccharidreste (z.B. malonyliert)
- R₄, R₅: -COOH, -Carbonsäureester
sowie zumindest ein weiteres Antioxidans und / oder Vitamin.

In weiteren Versuchen hat sich gezeigt, dass Verbindungen der Formel I bzw. NCCs sowohl pharmakologisch, dermatologisch oder kosmetisch eingesetzt werden können.

Besonders günstig ist es dabei, wenn R₁ gleich Vinyl (-CH=CH₂) oder -CHOH-CH₂OH, R₂ gleich -H oder -OH, R₃ gleich -H oder -OH oder ein Saccharidrest und R₄ bzw. R₅ gleich -COOH oder -COOCH₃ ist.

Weiters ist vorgesehen, dass die Zubereitung zumindest eine Verbindung der Formel I in einer Menge größer 0,001 % enthält.

Eine solche Zubereitung, insbesondere zum Schutz von Zellen gegen oxidativen Stress zur Verzögerung oder Verringerung der Membranoxidation ist dann besonders günstig, wenn die Antioxidantien und/oder Vitamine ausgewählt sind aus Vitamin A-Palmitat, Vitamin C und dessen Derivate, Tocopherol, Tocopherolacetat, Nikotinsäure, Pantothensäure und Biotin. Es wäre beispielsweise denkbar, ein Vitaminpräparat, z.B. in der Form einer Kapsel oder eines Getränkes, zuzubereiten.

In einer weiteren bevorzugten Ausführungsvariante ist vorgesehen, dass die Zubereitung einen oder mehrere UV-Filter enthält. In diesem Fall wäre es vorteilhaft, wenn die Zubereitung ein kosmetisches Mittel ist, vorzugsweise eine Hautcreme oder ein Sonnenschutzmittel.

Aufgrund weiterer Untersuchungen hat es sich ergeben, dass die Verbindung der Formel I physiologische Wirkungen aufweist. Daher ist es günstig, wenn die Zubereitung ein Arzneimittel, insbesondere ein Arzneimittel geeignet zur Prophylaxe und/oder Behandlung bei Organtransplantationen oder Herzattacken ist.

In diesem Fall wäre es außerdem denkbar, wenn die Zubereitung ein Hautbehandlungsmittel ist. Sowohl für die Erstellung eines Hautbehandlungsmittels oder einer Hautcreme oder Sonnenschutzmittels ist vorgesehen, dass die Zubereitung einen hautverträglichen Träger und/oder einen oder mehrere Wirkstoffe mit hautpflegender und/oder entzündungshemmender Wirkung enthält. Eine besondere Eignung als Pflege-/Sonnenschutzmittel, Hautcreme oder Arzneimittel zur Behandlung von Hautkrankheiten leitet sich u.a. aus den hervorragenden UV-Absorptionseigenschaften von NCCs ab. Dadurch wird schädliche UV-A und UV-B-Strahlung in unschädliche Wärmestrahlung umgewandelt.

Für eine Zubereitung als Arzneimittel ist es günstig, wenn diese in einer galenischen Form, vorzugsweise als Creme, Kapsel, Gel, Tablette vorliegt.

Weiters ist vorgesehen, dass die Zubereitung ein oder mehrere weitere Antioxidatien enthält. Die Kombination mehrerer Antioxidantien verbessert insgesamt die antioxidative Wirkung und weist den Synergieeffekt auf, dass in Situationen, bei denen ein Antioxidanz nicht wirksam ist, die antioxidative Wirkung des zweiten Antioxidans ergänzend "eingreift".

Weiters ist vorgesehen, dass die Zubereitung ein Nahrungsergänzungsmittel ist, vorzugsweise in einem Vitaminpräparat.

Augrund der hervorragenden Eigenschaften nicht nur als Antioxidationsmittel sondern auch der sehr guten Membrangängigkeit, da NCCs sowohl in wässrigen Lösungsmittel als auch in organischen Lösungsmitteln gut lösbar sind, ist es besonders geeignet, wenn eine solche Zubereitung ein Konservierungsmittel, vorzugsweise ein Nahrungskonservierungsmittel ist.

Ein weiterer Aspekt der Erfindung ist die Verwendung der genannten Zubereitungen als Konservierungsmittel, als Arzneimittel, als Nahrungsergänzungsmittel, als dermatologisches Mittel, vorzugsweise als Sonnenschutzmittel oder Hautcreme usw.

In einem weiteren Aspekt betrifft die Erfindung aufgrund der hervorragenden Eigenschaften die folgenden Punkte: 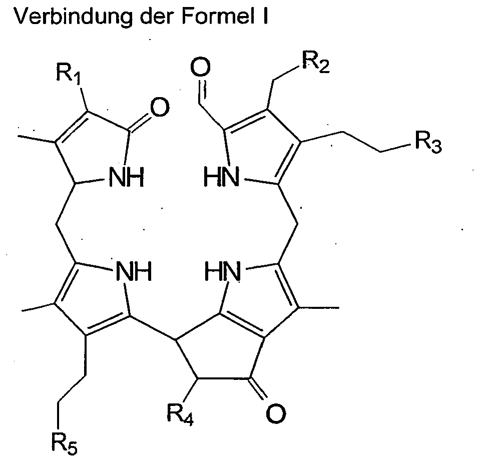 oder ein pharmakologisch, dermatologisch oder kosmetisch verträgliches Salz oder Derivat davon, wobei R₁ bis R₅ ausgewählt ist aus den Resten
- R₁: -Alkyl, -Vinyl, -CHOH-CH₂OH
- R₂: -H, -OH, -OAlkyl, -OAcyl, Saccharidreste, modifizierte Saccharidreste (z.B. malonyliert)
- R₃: -H, -OH, -OAlkyl, -OAcyl, modifizierte -OAcyl (z.B. malonyliert), Saccharidreste, modifizierte Saccharidreste (z.B. malonyliert)
- R₄, R₅: -COOH, -Carbonsäureester
als Konservierungsmittel, vorzugsweise als Nahrungskonservierungsmittel;
zur Verwendung als Arzneimittel;
als antibakterielles Mittel;
als fungizides Mittel;
als Nahrungsergänzungsmittel.
Nach bisherigem Erkenntnisstand kann davon ausgegangen werden, dass NCCs eine Fülle solcher und noch anderer Funktionen übernehmen können.

Es ist außerdem im Rahmen der Erfindung, die Verwendung einer Verbindung der Formel I zur Herstellung einer kosmetischen Zubereitung, einer pharmazeutischen Zubereitung, eines Nahrungsmittels und/oder eines Nahrungsergänzungsmittels vorgesehen. In diesem Fall ist es vorgesehen, die Verbindung zum Schutz gegen oxidativen Stress sowie zur Bekämpfung von Allergien, Entzündungen und/oder Irritationen, vorzugsweise Hautirritationen, zu verwenden.

Im Folgenden werden spezielle physiologische Eigenschaften von Clorophyllkataboliten, insbesondere im Zusammenhang mit Krebszellen, erläutert. Es konnte gezeigt werden, dass NCCs eine Zellzyklusblockade und Apoptose menschlicher Krebszellen verursachen kann. Es wurden drei humane Tumorzelllinien verschiedener Organe verwendet, und zwar Kolortkrebszellen der Linie SW480, Brustkrebszellen der Linie MDAMB231 und T-Zellen, Leukämiezellen der Linie CCRF-CEM.

Kolorektaler Krebs (CRC) ist das dritthäufigste Malignom weltweit. Das jährliche Vorkommen von CRC in Nordamerika und Europa beträgt ca. 30 - 50/100 000 Bewohner. Im Jahr 2000 war die Schätzung der American Cancer Society, dass es ca. 145 000 neue Fälle und ca. 56 000 Tote durch CRC in den Vereinigten Staaten gab. Als Todesursache liegt CRC sowohl bei Frauen als auch bei Männern an dritter Stelle, und zwar jeweils hinter Lungenkrebs und Brustkrebs bzw. Lungen- und Prostatakrebs. Das durchschnittliche Risiko, an CRC zu erkranken, beträgt während der Lebensspanne etwa 6%.

Unkontrollierte Zellproliferation ist ein Kennzeichen aller Krebsarten, daher wird Blockade des Zellzyklus als effizienteste Strategie zur Eliminierung von Krebszellen erachtet. Derzeit sind verschiedene Zellzyklusinhibitoren bekannt, die als Therapeutika gegen Krebs in präklinischen und klinischen Studien verwendet werden. Der Zellzyklus in Eukarionten wird zumindest teilweise von einer Familie von Proteinkinasenkomplexen kontrolliert, wobei jeder dieser Komplexe aus einer katalytischen Untereinheit, der so genannten zyklinabhängigen Kinase (CDK, Cyclin-Dependent-Kinase), und seiner essentiellen regulatorischen Untereinheit, dem Zyklin, besteht. Diese Komplexe werden zu spezifischen Zeitpunkten während eines jeden Zellzyklus aktiviert. Sie können jedoch auch durch exogene Faktoren reguliert werden. Der Zyklin-CDK-Komplex kann durch Bindung einer bestimmten Klasse von Proteinen, bekannt unter der Bezeichnung Zyklinkinaseinhibitoren (CKI), inhibiert werden. Antikrebsmittel können einen oder mehrere der regulatorischen Schritte im Zellzyklus beeinflussen, was in einer Blockade der Zellzyklusprogression mündet, wodurch das Wachstum und die Proliferation der Krebszellen reduziert wird. Eine Zellzyklusblockade kann zu einem programmierten Zelltod (d.h. zur Apoptose der Krebszelle) führen. Die Eigenschaft von Tumorzellen, der Apoptose zu entgehen, ist eine wichtige Eigenschaft in der Resistenz zu herkömmlichen therapeutischen Maßnahmen.

In der vorliegenden Studie konnte gezeigt werden, dass NCC-1 eine Disregulation des Zellzyklus und eine Induktion von Apoptose an verschiedenen menschlichen Krebszellen bewirkt.

Verwendete Materialen und Methoden:
Reagenzien: Der Chlorophyllkatabolit NCC-1 in einer Reinheit von über 95% wurde nach dem zuvor beschriebenen Reinigungsverfahren aus den seneszenten Blättern von Cercidiphyllum japonicum gewonnen. Eine Stammlösung von NCC-1 wurde durch Auflösen von 0,5g mit 70mg KH₂PO₄ in einer minimalen Menge vom Dulbeccos PPS-Puffer (erhalten von PAA Laboratories GmbH, Pasching, Austria) gelöst. Annexin-V APC und Propidiumjodid, Annexin-V Fitc und 7-AAD Färbelösung wurden von BD Farmingen (Heidelberg, Deutschland) und Sigma Aldrich (Deutschland) erhalten.

Zellkultur: Die menschliche Kolonkarzinomzelllinie SW480 stammte von der deutschen Sammlung von Mikroorganismen und Zellkulturen (Braunschweig, Deutschland). MDAMB231 und CCRF-CEM stammten von American Type Culture Collection (Rockville, MD). Die SW480 Zellen wurden in Dulbeccos modifizierten Eagles-Medium (DMEM; Sigma Alrich Chemie, Steinheim, Deutschland) kultiviert; MDAMB231-Zellen wurden in MEM (PAA Laboratories GmbH Pasching, Österreich) kultiviert. CCRF-CEM-Kulturen wurden in RPMI 1640 (PAA Laboratories) kultiviert. Allen Medien wurden 2 mM L-Glutamin, 10% FCS (fötales Kälberserum) und 1% Penicillin-Streptomycin versetzt. Die Zellen wurden unter Standardzellkulturbedingungen bei 37 °C und 5% CO₂ in feuchter Umgebung gehalten.

Behandlung von Zellen: Die Stammlösung von NCC-1 wurde durch ein 0,22-µm-Filter (Millex®-GS, Millipor, Carrigtwohill, Co. Cork, Irland) in ein steriles Gefäß filtriert und mit dem Zellkulturmedium verdünnt. Die endgültige Konzentration an NCC-1 betrug zwischen 50 µM und 200 µM. SW480 und MDAMB231 wurden auf 70 bis 80% Konfluenz in "6-Well-Platten" kultiviert (BD Biosciences) und wurden dann mit NCC-1 (1 000, 800, 600, 400, 200 µM/L Medium) für 24, 48 und 72 Stunden in vollständigem Zellkulturmedium behandelt. CCRF-CEM (300 000 Zellen/ml) wurden mit NCC-1 (800, 400, 200, 100 und 50 µM/L Medium) behandelt.

³H-Thymidin-Inkoporationsuntersuchungen: MDAMB231-Zellen (10 000 Zellen/Loch) wurden in flachbödige "96-Well-Mikrotiterplatten" in 200 µL an Wachstumsmedium mit 5% FCS gegeben und diese wurden mit Medien an unterschiedlichen Konzentrationen von NCC-1 (800, 1 200, 1 600, 2 000 µM/L) oder Medium alleine kultiviert. Die Zellen wurden für 18 Stunden mit NCC-1, kultiviert. Anschließend wurde 1 µCi an ³H-Thymidin hinzu gegeben, gefolgt von einer Inkubation bei 37 °C in einer feuchten 5% CO₂-Atmosphäre für weitere sechs Stunden; Lagerung erfolgte bei -70 °C über Nacht. Am Folgetag wurde die gefrorene Platte bei 37 °C getaut und die Inkorporation an ³H-Thymidin durch Flüssigszintillationsmessung ermittelt. Jede Probenbestimmung erfolgte vierfach.

Bestimmung der Apoptose mittels Durchflusszytometrie: CCRF-CEM wurde in 6-Well-Platten herangezüchtet (300 000 Zellen/ml) und mit NCC-1 (800, 400, 200, 100 und 50 µM/L) für 24 Stunden, 48 Stunden und 72 Stunden behandelt. Die Zellen wurden zweimal mit kaltgestelltem PBS gespült und im Bindungspuffer resuspendiert (1 mM Hepes/NaOH, pH 7,4, 140 mM NaCl, 2,5 mM CaCl₂). 5 µL Annexin-V-FITC (BD Farmingen) und 5 µL 7-Amino-Aktinomyzin D (7-ADD, BD Farmingen) wurden zugegeben und für 15 Minuten bei Raumtemperatur unter Lichtausschluss inkubiert.

DNA-Zellzyklusanalyse: Adhärente Zellen (SW480 und MDAMB231; 70 bis 80% konfluent) wurden mit NCC-1 (1 000, 800, 600, 400, 200 µM/L) behandelt. Die suspendierten Zellen CCRF-CEM wurden in 800, 400, 200, 100 und 50 µM NCC-1/l in vollständigem Medium für 24, 48, und 72 Stunden behandelt. SW480 und MDAMB231 wurden danach mit Trypsin verdaut; zweimal mit kaltem PBS gewaschen und zentrifugiert. CCRF-CEM-Zellen wurden zweimal mit kaltem PBS gewaschen und zentrifugiert. Das Zellpellet wurde in 50 µL Nicolettipuffer (die Endkonzentration an Propidiumjodid betrug 50 µg/mL) resuspendiert, anschließend für zwei Stunden bei 4 °C in der Dunkelheit inkubiert und schlussendlich mittels Durchflusszytometrie analysiert.

Vorbereitung von Zelllysaten und Western-Blot-Analyse: Die CCRF-CEM-Zellen wurden jeweils nach 4, 8m 24 und 48 Stunden nach erfolgter NCC-1-Behandlung (200 µM; wie oben beschrieben) geerntet und mit kalten PBS gewaschen. Die Zellen wurden in eisgekültem Lysepuffer (50 mM/L Tris-HCl, 100 mM/L NaCl, 50 mM/L NaF, 5 mM/L EDTA, 40 mM β-Glycerophosphat, 1% Triton X-100) inkubiert und mit frischem Halt Proteaseinhibitor-Cocktail (Pierce, Illinois, USA) und 200 µM/L Na₃VO₄ versetzt. Die Zellen wurden während mehrerer Einfrier- und Auftauzyklen (abwechselndes Kühlen mittels flüssigem Stickstoff und Erwärmen auf 37 °C) lysiert. Das Lysat wurde durch Zentrifugieren bei 14 000 g für 15 Minuten bei 4 °C gesäubert, der Überstand (das gesamte Zelllysat) wurde gesammelt aliquotiert und bei -70 °C gelagert. Der Proteingehalt in den Lysaten wurde durch Bio-Radprotein-Essay (Bio-Rad Laboratories) nach dem Protokoll des Herstellers analysiert.

Für Western-Blot-Analyse wurden 50 µg Protein auf 8 bis 12% SDS-PAGE-Gel aufgelöst und auf eine Nitrozellulosemembran transferiert. Die nicht spezifischen Stellen wurden blockiert, indem der Blot mit 5% fettfreier Trockenmilch in Puffer (enthaltend 10 mM/L Tris, 100 mM/L NaCl, 0,1% Tween 20) für eine Stunde bei Raumtemperatur oder über Nacht bei 4 °C inkubiert wurde. Anschließend wurde der Blot mit Waschpuffer (10 mM/L Tris, 100 mM/L NaCl, 0,1% Tween 20) dreimal für jeweils zehn Minuten gewaschen und anschließend erfolgte die Inkubation über Nacht mit einem geeignetem Primärantikörper spezifisch für das zu bestimmende Protein. Die Antikörper wurden in Konzentrationen wie vom Hersteller angegeben verwendet. Der Blot wurde für zweimal je 20 Minuten gewaschen, gefolgt von Inkubation mit dem entsprechenden Sekundärantikörper, der Meerrettichperoxidase (HRPO, horseradish peroxidase) konjugiert ist (Amersham Life Science Inc., Arlington Heights, IL) bei einer 1:2 000 Verdünnung für eine Stunde bei Raumtemperatur. Der Blot wurde anschließend in Waschpuffer zweimal für jeweils zehn Minuten und viermal für jeweils fünf Minuten gewaschen. Der Proteingehalt wurde mittels Chemielumineszenz mit dem Enhanced Chemiluminescence Kit (Amersham Life Science) und Autoradiographie mit XAR-5-Film (Amersham Life Science) detektiert. Für jeden Immunoblot wurde derselbe Proteingehalt durch "Stripping" des Blots und Rückuntersuchung mit β-Aktin-Antikörpern bestätigt.

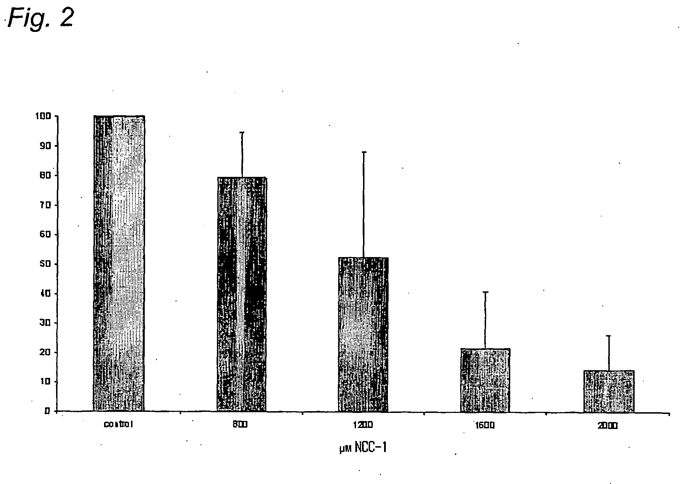

Anhand der Fig. 2 bis 6 und der Figurenbeschreibung werden im Folgenden die Ergebnisse und Resultate der krebstherapierenden Wirkung von NCC-1 beschrieben. Es wurden drei humane Zelllinien verschiedener Organe verwendet, und zwar Kolonkrebszellen der Linie SW 480, Brustkrebszellen der Linie MDAMB231 und T-Zellen, Leukämiezellen der Linie CCRF-CEM.

Im ersten Teil der Experimente wurde untersucht, ob NCC-1-Behandlung der Zelllinien einen antiproliferativen Effekt auf die Krebszellen hat. Zu diesem Zweck wurde der In-Vitro-Effekt von NCC-1 auf die Tumorzellenproliferation mittels Tritiumthymidin-Inkorporierungsassay untersucht. Es konnte gezeigt werden, dass NCC-1-Behandlung dreier verschiedener Krebszelllinien in einer dosisabhängigen Verringerung des Wachstums aller Zelltypen resultierte (siehe hierzu Fig. 2; die Daten für SW 480 und CCRF-CEM sind übersichtlichkeithalber nicht gezeigt; das Diagramm zeigt die % Zellwachstum in Abhängigkeit von der NCC-1 Konzentration: Kontrolle = 0, 800, 1200, 1600, 2000 µM). Mit steigender Dosis konnte gegenüber einer Kontrolle mit einem relativen Zellwachstum von 100% eine Verringerung auf ca. 15% mit einer 2 000 µM/L NCC-1-Behandlung erzielt werden. Die Balken entsprechen dabei dem Mittelwert von drei verschiedenen Experimenten, die Standardabweichung ist mittels der Fehlerbalkenstriche verdeutlicht.

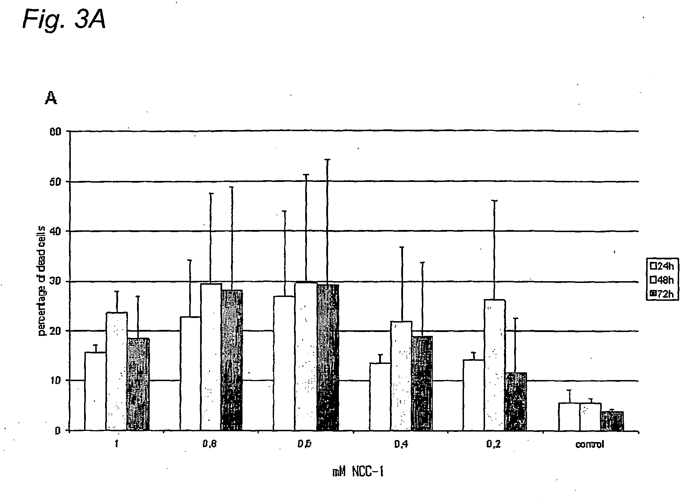

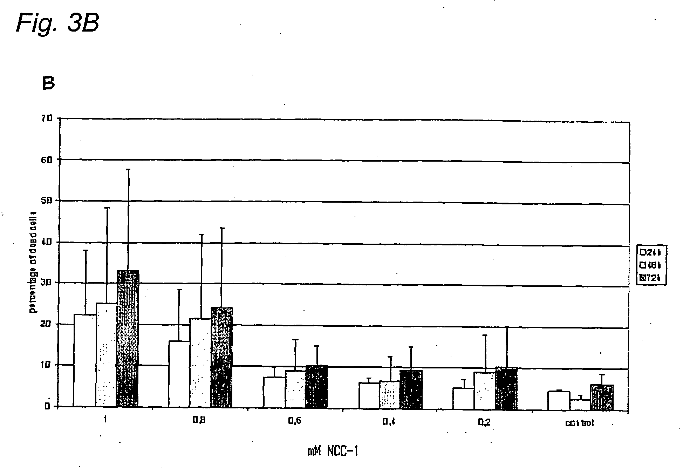

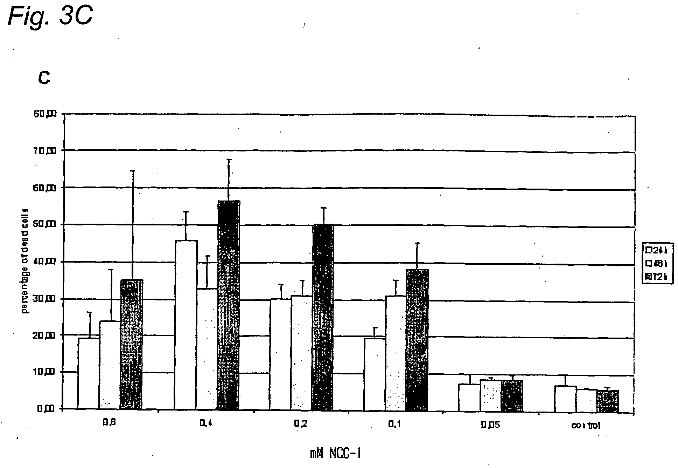

In einem nächsten Schritt wurde der Einfluss von NCC-1 auf den Zellzyklus von Krebszellen untersucht (Fig. 3A bis 3C; aufgetragen sind die Prozent an toten Zellen gegen die NCC-1 Konzentration, die 3 Balken stehen für 24, 48 und 72 Stunden). Die wachsenden Zellen wurden mit NCC-1 behandelt, und zwar für 24, 48 und 72 Stunden, der DNA-Zellzyklus wurde wie zuvor beschrieben analysiert. Gegenüber einer Kontrolle konnte gezeigt werden, dass mit Zugabe von NCC-1 die Anzahl an toten Zellen (in Prozent) gegenüber der Kontrolle erheblich anstieg. In der Fig. 3A ist hierzu das Experiment für MDAMB231-Zellen, in der Fig. 3B für SW480-Zellen und in der Fig. 3C für CCRF-CEM-Zellen gezeigt. Die Balken zeigen den Mittelwert drei verschiedener Experimente jeweils in Duplikaten ausgeführt, wobei die Standardabweichung ebenfalls angezeigt ist.

Wie in den Fig. 3A bis 3C gezeigt werden konnte, ergibt sich dass NCC-1 den Zelltod in verschiedenen Krebszelltypen induziert. Mikroskopische Untersuchungen zeigten unterschiedliche Zellmorphologien bei unterschiedlichen Konzentrationen von NCC. Bei niedrigen Konzentrationen verursachte die NCC-1-Behandlung der Krebszellen Bläschenbildung an den Zellen, was ein typisches Zeichen für Apoptose ist, während sich bei höheren NCC-1-Konzentrationsbehandlungen die Zellen aufblähten, was ein Anzeichen für Nekrose ist. Als Ergebnis dieser Voruntersuchungen wurde anschließend untersucht, ob die unterschiedlichen morphologischen Differenzen mit unterschiedlichen Typen von Zelltod korrespondierten.

Apoptotischer Zelltod ist die Folge einer Serie von präzise regulierten Ereignissen, welche in Tumorzellen häufig abgewandelt sind. Dies bietet eine Möglichkeit für selektive klinische Intervention, um einen programmierten Zelltod der Krebszellen zu induzieren, idealerweise ohne die normalen Zellen zu beeinflussen. Apoptose ist ein physiologischer Prozess, der die Eliminierung von Zellen mit DNA-Beschädigung involviert und er stellt eine selbständige Form von Zelltod dar, die sich von nekrotischem Zelltod unterscheidet. Folglich sind Stoffe, die Apoptose regeln oder beeinflussen können, in der Krebstherapie einsetzbar.

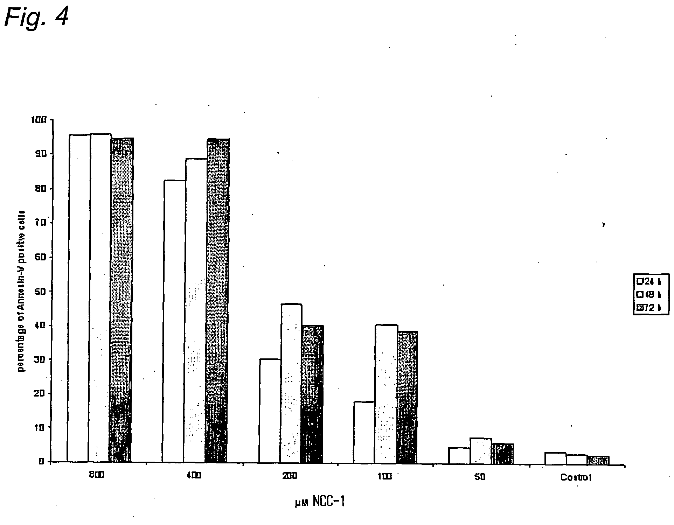

Wie in der Fig. 4 (aufgetragen sind die Prozent Annexin-V-positiven Zellen gegen die NCC-1 Konzentration, die drei Balken stehen für 24, 48, 72 Stunden) gezeigt, resultiert bei CCRF-CEM-Zellen die Behandlung mit NCC-1 in einer dosisabhängigen Induktion von Apoptose. Gegenüber den Kontrollen konnte bei Zugabe von 400 µM NCC-1 oder mehr ein beinahe 100%ige Annexin-V-positives Ergebnis erzielt werden.

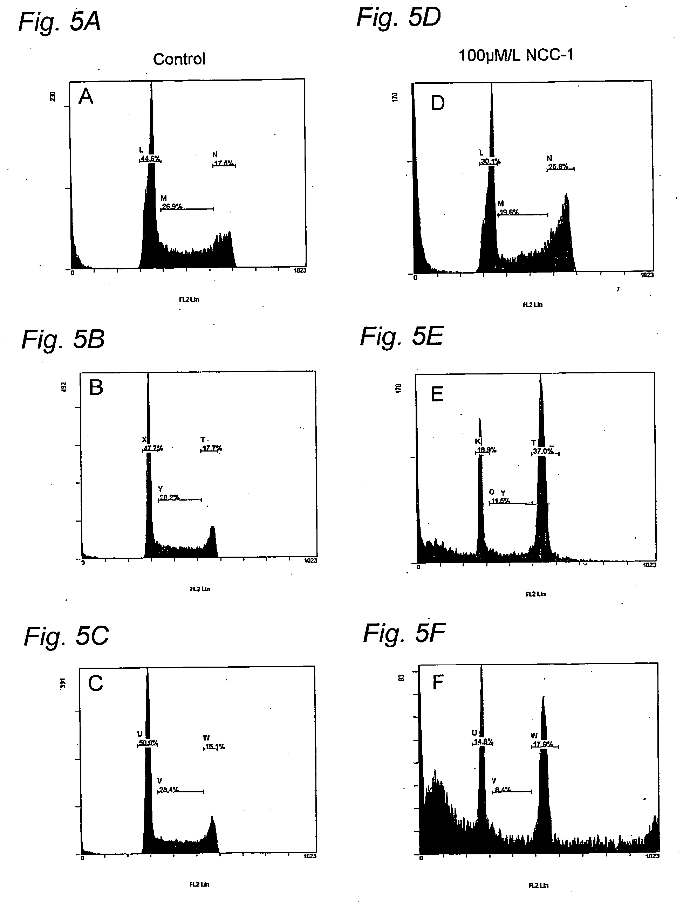

In Zellzytrometrieanalysen (FACS) konnte gezeigt werden, dass in allen drei Zelllinien durch NCC-1-Behandlung ein dosisabhängiger G2-Arrest erfolgt (Fig. 5A-5F; die wachsenden Zellen wurden sowohl mit reinem Zellkulturmedium 24 Stunden (5A), 48 Stunden (5B) und 72 Stunden (5C) als auch mit 100 µM NCC-1 24 Stunden (5D), 48 Stunden (5E) und 72 Stunden (5F) behandelt.). Dies ist ein Hinweis, dass die Induktion des Apoptose vom Zellzyklus abhängig ist. Um diese Hinweise zu überprüfen, wurde in einer weiteren Serie von Experimenten bestätigt, dass die NCC-1 verursachte Apoptose der CCRF-CEM-Zellen durch Zellzyklusblockade verursacht wird. Diese Untersuchung ist besonders wichtig, da die molekularen Analysen an menschlichem Krebs gezeigt haben, dass Zellzyklusregulatoren häufig in bösartigen Erkrankungen mutiert sind. In den Fig. 5A bis 5F wird der Effekt der NCC-1-Behandlung auf den Zellzyklus in den CCRF-CEM-Zellen gezeigt. Die wachsenden Zellen wurden in Medium allein für 24 Stunden (A), 48 Stunden (B) und 72 Stunden (C) bzw. mit NCC-1 (100 µmol/l) für 24 Stunden (D), 48 Stunden (E) und 72 Stunden (F) behandelt. In den Darstellungen ist die Zahl als Funktion der Größe dargestellt. Deutlich erkennbar ist eine Verschiebung zu unterschiedlichen Größen.

In einem nächsten Schritt wurde der Einfluss von NCC-1 auf die CKI-Zyklin-CDK-Maschinerie in G2/M-Phase Zellzyklus-Arrest in menschlichen Krebszellen untersucht. Das Durchschreiten der Zellen durch den Zellzyklus in Eukarionten wird durch die oben erwähnte Familie von Proteinkinasekomplexen koordiniert. Jeder Komplex ist, wie bereits erwährt, zumindest aus der regulatorischen Untereinheit Zyklin, das auf die katalytische CDK Untereinheit bindet, zusammengesetzt, um den aktiven Zyklin-CDK-Komplex zu bilden. Solche Komplexe werden bei verschiedenen Zeitpunkten nach spezifischen Intervallen während des Zellzyklus aktiviert und sie können auch durch exogene Faktoren reguliert werden. In transformierten Zellen ist die Zellprogression ein mitogener Signalabhängiger oder mitogener Signalunabhängiger Prozess. CDK-Aktivität wird zusätzlich durch kleine Proteine, bekannt als CKIs, reguliert. CKIs beinhalten die p21/WAF1 und p27/KIP1-Proteinfamilien. Deshalb wurde die Modellierung in Zellzyklusregulatorischen Ereignissen im Zusammenhang mit der G2/M-Phase als Mechanismus der NCC-1 vermittelten Zellzyklusdysregulation und Apoptose in menschlichen CCRF-CEM-Zellen untersucht.

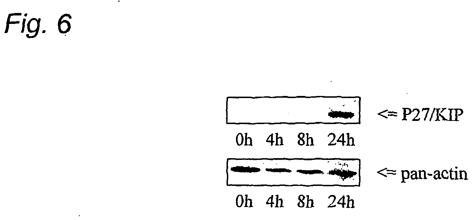

Mittels Western-Blot-Analyse (Fig. 6) konnte für die NCC-1-Behandlung (200 µMol/L für 4, 8 und 24 Stunden, Kontrolle wurde mit Medium allein kultiviert) von CCRF-CEM-Zellen gezeigt werden, dass eine signifikante Hochregulierung des CKI p27/KIP1 Protein erfolgte. In jüngeren Studien wurde die kritische Rolle von p27/KIP1 bei der Apoptose und der Zellzyklusprogression durch die G2-Phase gezeigt.

Die gezeigten experimentellen Daten belegen einwandfrei, dass NCCs eine therapeutische Wirkung bei Krebserkrankungen besitzen und ihnen präventive Wirkung auf solche Erkrankungen zukommt. Einer gezielten Zugabe von NCCs zu dermatologischen Produkten, Nahrungsmitteln kann daher neben einem konservierenden Effekt auch noch eine präventive Funktion für bestimmte Erkrankungen zukommen.

## Patentansprüche

1. Zubereitung, enthaltend zumindest eine Verbindung der Formel I 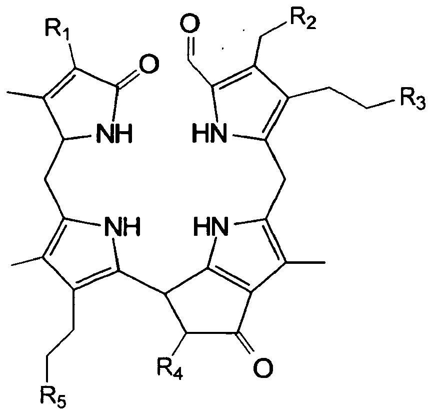 oder ein pharmakologisch, dermatologisch oder kosmetisch verträgliches Salz oder Derivat davon, wobei R₁ bis R₅ ausgewählt ist aus den Resten
R₁ -Alkyl, -Vinyl, -CHOH-CH₂OH
R₂ -H, -OH, -OAlkyl, -OAcyl, Saccharidreste, modifizierte Saccharidreste,
R₃ -H, -OH, -OAlkyl, -OAcyl, modifizierte -OAcyl,
Saccharidreste, modifizierte Saccharidreste, R₄, R₅ -COOH, - Carbonsäureester,
sowie zumindest ein weiteres Antioxidans und / oder Vitamin.

2. Zubereitung nach Anspruch 1, **dadurch gekennzeichnet, dass** R₁ gleich Vinyl (-CH=CH₂) oder -CHOH-CH₂OH, R₂ gleich -H oder -OH, R₃ gleich -H oder -OH oder ein Saccharidrest, R₄ gleich - COOH oder COOCH₃ und R₅ gleich -COOH ist.

3. Zubereitung nach einem der Ansprüche 1 oder 2, **dadurch gekennzeichnet, dass** die Zubereitung zumindest eine Verbindung der Formel I in einer Menge größer 0,001% enthält.

4. Zubereitung nach einem der Ansprüche 1 bis 3, **dadurch gekennzeichnet, dass** die Antioxidantien und/oder Vitamine ausgewählt sind aus der Gruppe aus Vitamin A, Vitamin A-Palmitat, Vitamin C und dessen Derivate, Tocopherol, Tocopherolacetat, Nikotinsäure, Pantothensäure und Biotin.

5. Zubereitung nach einem der Ansprüche 1 bis 4, **dadurch gekennzeichnet, dass** die Zubereitung einen oder mehrere UV-Filter enthält.

6. Zubereitung nach einem der Ansprüche 1 bis 5, **dadurch gekennzeichnet, dass** diese ein kosmetisches Mittel ist.

7. Zubereitung nach einem der Ansprüche 1 bis 5, **dadurch gekennzeichnet, dass** die Zubereitung ein Arzneimittel ist.

8. Zubereitung nach einem der Ansprüche 1 bis 5, **dadurch gekennzeichnet, dass** diese ein Hautbehandlungsmittel ist.

9. Zubereitung nach einem der Ansprüche 1 bis 8, **dadurch gekennzeichnet, dass** sie einen hautverträglichen Träger und/oder einen oder mehrere Wirkstoffe mit hautpflegender und/oder entzündungshemmender Wirkung enthält.

10. Zubereitung nach einem der Ansprüche 7 bis 9, **dadurch gekennzeichnet, dass** sie in einer galenischen Form vorliegt.

11. Zubereitung nach einem der Ansprüche 1 bis 4, **dadurch gekennzeichnet, dass** diese ein Nahrungsergänzungsmittel ist.

12. Zubereitung nach einem der Ansprüche 1 bis 4, **dadurch gekennzeichnet, dass** diese ein Konservierungsmittel ist.

13. Zubereitung nach einem der Ansprüche 1 bis 5 und 7 bis 10 zur Verwendung als Arzneimittel.

14. Zubereitung nach einem der Ansprüche 1 bis 4 zur Verwendung als Antioxidationsmittel.

15. Verwendung einer Zubereitung nach einem der Ansprüche 1 bis 5 als Konservierungsmittel.

16. Verwendung einer Zubereitung nach einem der Ansprüche 1 bis 4 und 11 bis 12 als Nahrungsergänzungsmittel.

17. Zubereitung nach einem der Ansprüche 1 bis 5 oder 9 und 10 zur Verwendung als dermatologisches Mittel.

18. Verbindung der Formel I 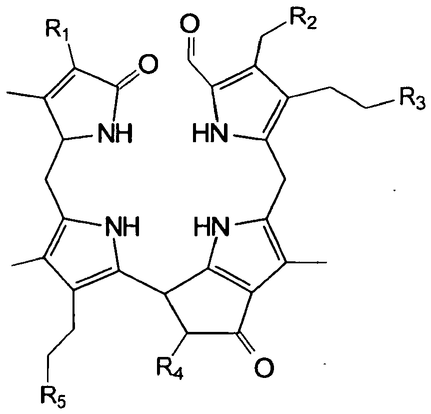 oder ein pharmakologisch, dermatologisch oder kosmetisch verträgliches Salz oder Derivat davon, wobei R₁ bis R₅ ausgewählt ist aus den Resten
R₁ -Alkyl, -Vinyl, -CHOH-CH₂OH
R₂ -H, -OH, -OAlkyl, -OAcyl, Saccharidreste, modifizierte Saccharidreste,
R₃ -H, -OH, -OAlkyl, -OAcyl, modifizierte -OAcyl,
Saccharidreste, modifizierte Saccharidreste, R₄, R₅ -COOH, - Carbonsäureester
zur Verwendung als Arzneimittel.

19. Verbindung der Formel I 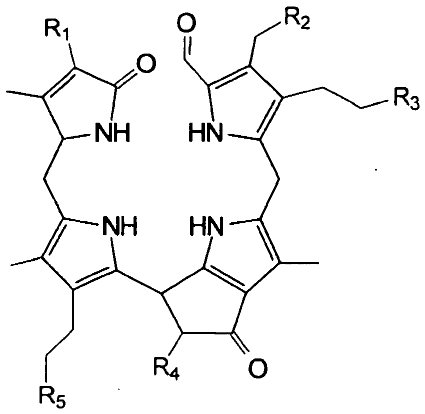 oder ein pharmakologisch, dermatologisch oder kosmetisch verträgliches Salz oder Derivat davon, wobei R₁ bis R₅ ausgewählt ist aus den Resten
R₁ -Alkyl, -Vinyl, -CHOH-CH₂OH
R₂ -H, -OH, -OAlkyl, -OAcyl, Saccharidreste, modifizierte Saccharidreste,
R₃ -H, -OH, -OAlkyl, -OAcyl, modifizierte -OAcyl,
Saccharidreste, modifizierte Saccharidreste, R₄, R₅ -COOH, Carbonsäureester'
als Antioxidationsmittel.

20. Verbindung der Formel I 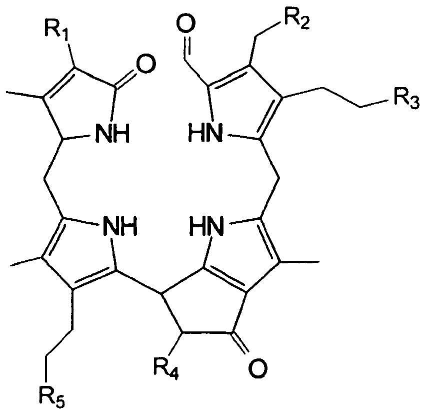 oder ein pharmakologisch, dermatologisch oder kosmetisch verträgliches Salz oder Derivat davon, wobei R₁ bis R₅ ausgewählt ist aus den Resten
R₁ -Alkyl, -Vinyl, -CHOH-CH₂OH
R₂ -H, -OH, -OAlkyl, -OAcyl, Saccharidreste, modifizierte Saccharidreste,
R₃ -H, -OH, -OAlkyl, -OAcyl, modifizierte -OAcyl,
Saccharidreste, modifizierte Saccharidreste, R₄, R₅ -COOH, **-** Carbonsäureester
als Konservierungsmittel.

21. Verbindung der Formel I 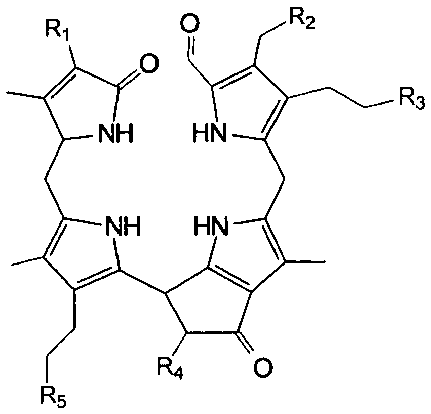 oder ein pharmakologisch, dermatologisch oder kosmetisch verträgliches Salz oder Derivat davon, wobei R₁ bis R₅ ausgewählt ist aus den Resten
R₁ -Alkyl, -Vinyl, -CHOH-CH₂OH
R₂ -H, -OH, -OAlkyl, -OAcyl, Saccharidreste, modifizierte Saccharidreste,
R₃ -H, -OH, -OAlkyl, -OAcyl, modifizierte -OAcyl,
Saccharidreste, modifizierte Saccharidreste, R₄, R₅ -COOH, - Carbonsäureester
als antibakterielles Mittel.

22. Verbindung der Formel I 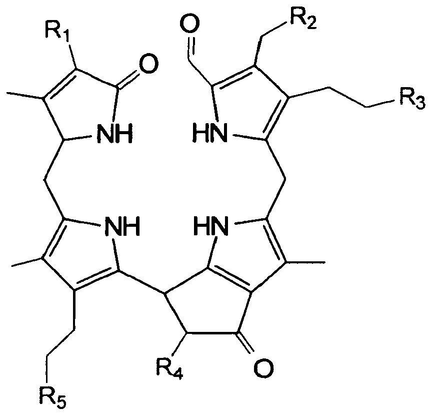 oder ein pharmakologisch, dermatologisch oder kosmetisch verträgliches Salz oder Derivat davon, wobei R₁ bis R₅ ausgewählt ist aus den Resten
R₁ -Alkyl, -Vinyl, -CHOH-CH₂OH
R₂ -H, -OH, -OAlkyl, -OAcyl, Saccharidreste, modifizierte Saccharidreste,
R₃ -H, -OH, -OAlkyl, -OAcyl, modifizierte -OAcyl,
Saccharidreste, modifizierte Saccharidreste, R₄, R₅ -COOH, - Carbonsäureester
als fungizides Mittel.

23. Verbindung der Formel I 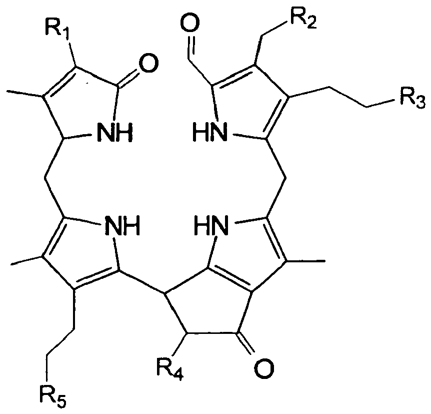 oder ein pharmakologisch, dermatologisch oder kosmetisch verträgliches Salz oder Derivat davon, wobei R₁ bis R₅ ausgewählt ist aus den Resten
R₁ -Alkyl, -Vinyl, -CHOH-CH₂OH
R₂ -H, -OH, -OAlkyl, -OAcyl, Saccharidreste, modifizierte Saccharidreste,
R₃ -H, -OH, -OAlkyl, -OAcyl, modifizierte -OAcyl,
Saccharidreste, modifizierte Saccharidreste, R₄, R₅ -COOH, - Carbonsäureester
als Nahrungsergänzungsmittel.

24. Verwendung einer Verbindung der Formel I 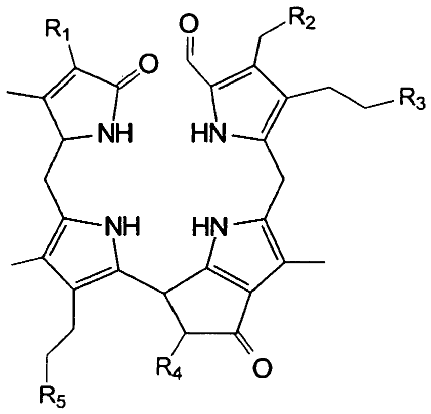 oder ein pharmakologisch, dermatologisch oder kosmetisch verträgliches Salz oder Derivat davon, wobei R₁ bis R₅ ausgewählt ist aus den Resten
R₁ -Alkyl, -Vinyl, -CHOH-CH₂OH
R₂ -H, -OH, -OAlkyl, -OAcyl, Saccharidreste, modifizierte Saccharidreste,
R₃ -H, -OH, -OAlkyl, -OAcyl, modifizierte -OAcyl,
Saccharidreste, modifizierte Saccharidreste, R₄, R₅ -COOH, - Carbonsäureester
zur Herstellung eines Arzneimittels zur Vorbeugung und/oder Behandlung von Krebserkrankungen.

25. Verwendung nach Anspruch 24, **dadurch gekennzeichnet, dass** die Krebserkrankung aus der Gruppe Hautkrebs, Brustkrebs, Lungenkrebs, Darmkrebs oder Leukämie stammt.

26. Verwendung einer Verbindung der Formel I 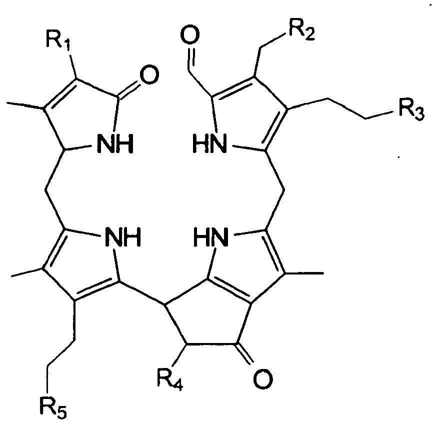 oder ein pharmakologisch, dermatologisch oder kosmetisch verträgliches Salz oder Derivat davon, wobei R₁ bis R₅ ausgewählt ist aus den Resten
R₁ -Alkyl, -Vinyl, -CHOH-CH₂OH
R₂ -H, -OH, -OAlkyl, -OAcyl, Saccharidreste, modifizierte Saccharidreste,
R₃ -H, -OH, -OAlkyl, -OAcyl, modifizierte -OAcyl,
Saccharidreste, modifizierte Saccharidreste, R₄, R₅ -COOH, - Carbonsäureester
zur Herstellung einer kosmetischen Zubereitung, einer pharmazeutischen Zubereitung, eines Nahrungsmittels und/oder eines Nahrungsergänzungsmittels.

27. Verwendung nach Anspruch 26 zum Schutz gegen oxidativen Stress sowie zur Bekämpfung von Allergien, Entzündungen und/oder Irritationen.

28. Verwendung nach Anspruch 26 zur Herstellung eines Konservierungsmittels.

29. Verfahren zur Gewinnung von nicht-fluoreszierenden Chlorophyllmetaboliten mit der chemischen Formel I 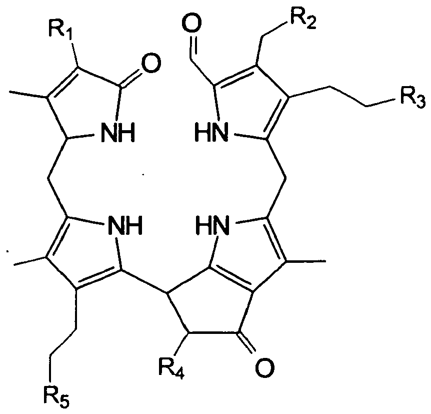 oder ein pharmakologisch, dermatologisch oder kosmetisch verträgliches Salz oder Derivat davon, wobei R₁ bis R₅ ausgewählt ist aus den Resten
R₁ -Alkyl, -Vinyl, -CHOH-CH₂OH
R₂ -H, -OH, -OAlkyl, -OAcyl, Saccharidreste, modifizierte Saccharidreste,
R₃ -H, -OH, -OAlkyl, -OAcyl, modifizierte -OAcyl,
Saccharidreste, modifizierte Saccharidreste, R₄, R₅ -COOH, - Carbonsäureester
sowie dessen Salze und Solvate aus pflanzlichem Material **gekennzeichnet durch** die Schritte:
• Extraktion des pflanzlichen Materials mit einem ersten Lösungsmittel oder Lösungsmittelgemisch mit einer Dielektrizitätskonstante von 5 bis 20 und einem Dipolmoment von 5 bis 10 × 10⁻³⁰ Cm zu einem Rohextrakt
• Filtration oder Chromatographie dieses Extraktes über Kieselgel
• Elution des Kieselgels mit einem zweiten Lösungsmittel oder Lösungsmittelgemisch mit größerer Polarität als das erste Lösungsmittel
• Abziehen des Lösungsmittels und gegebenenfalls Kristallisation des erhaltenen Rückstandes.

30. Verfahren nach Anspruch 29, **dadurch gekennzeichnet, dass** als erstes Lösungsmittel CH₂Cl₂, Methylacetat, Ethylacetat, t-Butanol, Ethylmethylketon oder Mischungen daraus verwendet werden.

31. Verfahren nach Anspruch 29 oder 30, **dadurch gekennzeichnet, dass** der Filtrationsschritt mit einer kieselgelgefüllten Chromatographiesäule durchgeführt wird.

32. Verfahren nach einem der Ansprüche 29 bis 31, **dadurch gekennzeichnet, dass** das Lösungsmittel für den Eluationsschritt ein Lösungsmittel größerer Polarität als das erste Lösungsmittel verwendet wird und aus der Gruppe Aceton, Ethylmethylketon, Methanol, Methanol-Wasser-Gemische, Methylacetat oder Mischungen davon oder Mischungen des ersten Lösungsmittels oder Lösungsmittelgemisches mit einem Lösungsmittel größerer Polarität besteht.

33. Verfahren nach einem der Ansprüche 29 bis 32, **dadurch gekennzeichnet, dass** vor dem Filtrationsschritt ein Reinigungsschritt des Rohextraktes mit einem nicht mischbaren Lösungsmittel bzw. einer nicht mischbaren wässrigen Lösung erfolgt.

34. Verfahren nach einem der Ansprüche 29 bis 33, **dadurch gekennzeichnet, dass** das Eluat mit einer wässrigen Lösung gewaschen wird.

35. Verfahren nach einem der Ansprüche 29 bis 34, **dadurch gekennzeichnet, dass** der Rückstand aus dem Verdampfungsschritt kristallisiert wird und anschließend filtriert und getrocknet wird.

36. Verfahren nach einem der Ansprüche 29 bis 35, **dadurch gekennzeichnet, dass** das Kieselgel eine Korngröße von unter 5000 aufweist.

37. Verfahren nach einem der Ansprüche 29 bis 36, **dadurch gekennzeichnet, dass** als pflanzliches Material Pflanzenteile verwendet werden, die vor der Reifung oder vor der Seneszenz grün sind.

38. Verfahren nach einem der Ansprüche 29 bis 37, **dadurch gekennzeichnet, dass** als pflanzliches Material im Wesentlichen senesziertes oder gereiftes Pflanzenmaterial verwendet wird.

39. Verfahren nach einem der Ansprüche 29 bis 38, **dadurch gekennzeichnet, dass** als Pflanzenmaterial frisch geerntetes, senesziertes oder gereiftes Pflanzenmaterial oder senesziertes oder gereiftes Pflanzenmaterial, das nach der Ernte tiefgekühlt (bei etwa -20°C oder darunter) gelagert wurde, verwendet wird.

40. Verfahren nach einem der Ansprüche 29 bis 39, **dadurch gekennzeichnet, dass** das Pflanzenmaterial aus Agrarpflanzen wie Ölraps, Klee, Gerste, Mais, Tabak, von Krautpflanzen und landwirtschaftlich produzierten Gemüsepflanzen wie Spinat, Brokkoli, Paprika, von Sträuchern und Laubbäumen wie Hamamelis, Ulmen, Platanen, Nussbäumen, *Cercidiphyllum japonicum* und von Nadelbäumen wie Lärchen stammt.

## Claims

1. Preparation containing at least one compound of formula I 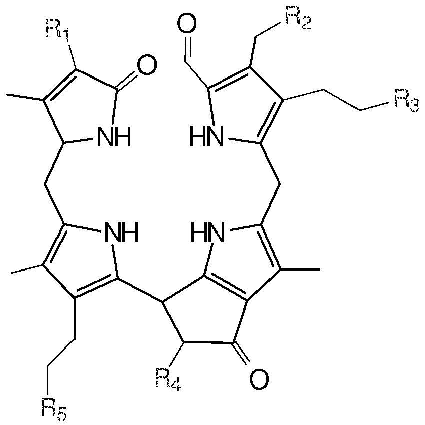 or a pharmacologically, dermatologically or cosmetically acceptable salt or derivative thereof, wherein R₁ to R₅ is selected from the radicals
R₁ -alkyl, -vinyl, -CHOH-CH₂OH
R₂ -H, -OH, -Oalkyl, -Oacyl, saccharide radicals, modified saccharide radicals,
R₃ -H, -OH, -Oalkyl, -Oacyl, modified -Oacyl, saccharide radicals, modified saccharide radicals,
R₄, R₅ -COOH, carboxylic acid ester, and
at least one further antioxidant and/or vitamin.

2. Preparation according to claim 1, wherein R₁ is vinyl (-CH=CH₂) or -CHOH-CH₂OH, R₂ is -H or -OH, R₃ is -H or -OH or a saccharide radical, R₄ is COOH or COOCH₃ and R₅ is -COOH.

3. Preparation according to claim 1 or claim 2, wherein the preparation contains at least one compound of formula I in a quantity greater than 0.001 %.

4. Preparation according to one of claims 1 to 3, wherein the antioxidants and/or vitamins are selected from vitamin A, vitamin A palmitate, vitamin C and its derivatives, tocopherol, tocopherol acetate, nicotinic acid, pantothenic acid, and biotin.

5. Preparation according to one of claims 1 to 4, wherein the preparation contains one or more UV filters.

6. Preparation according to one of claims 1 to 5, wherein it is a cosmetic agent.

7. Preparation according to one of claims 1 to 5, wherein the preparation is a medicinal product.

8. Preparation according to one of claims 1 to 5, wherein it is a skin treatment agent.

9. Preparation according to one of claims 1 to 8, wherein it contains a hypoallergenic support and/or one or more active ingredients with a skincare and/or anti-inflammatory action.

10. Preparation according to one of claims 7 to 9, wherein it is present in a galenic form.

11. Preparation according to one of claims 1 to 4, wherein it is a food supplement.

12. Preparation according to one of claims 1 to 4, wherein it is a preservative.

13. Preparation according to one of claims 1 to 5 and 7 to 10 for use as a medicinal product.

14. Preparation according to one of claims 1 to 4 for use as an antioxidant.

15. Use of a preparation according to one of claims 1 to 5 as a preservative.

16. Use of a preparation according to one of claims 1 to 4 and 11 to 12 as a food supplement.

17. Preparation according to one of claims 1 to 5 or 9 and 10 for use as a dermatological agent.

18. Compound of formula I 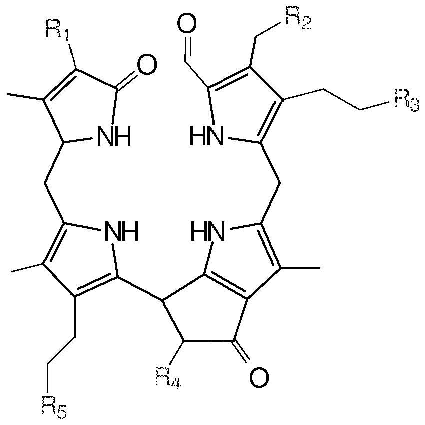 or a pharmacologically, dermatologically or cosmetically acceptable salt or derivative thereof, wherein R₁ to R₅ is selected from the radicals
R₁ -alkyl, -vinyl, -CHOH-CH₂OH
R₂ -H, -OH, -Oalkyl, -Oacyl, saccharide radicals, modified saccharide radicals,
R₃ -H, -OH, -Oalkyl, -Oacyl, modified -Oacyl, saccharide radicals, modified saccharide radicals,
R₄, R₅ -COOH, carboxylic acid ester
for use as a medicinal product.

19. Compound of formula I 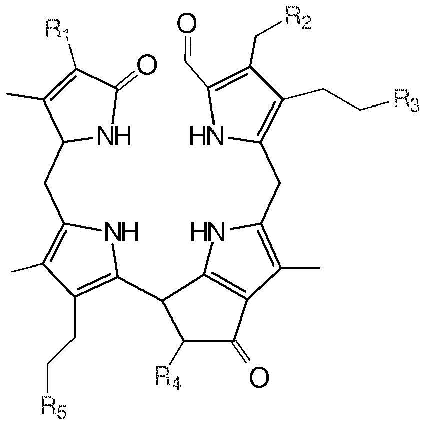 or a pharmacologically, dermatologically or cosmetically acceptable salt or derivative thereof, wherein R₁ to R₅ is selected from the radicals
R₁ -alkyl, -vinyl, -CHOH-CH₂OH
R₂ -H, -OH, -Oalkyl, -Oacyl, saccharide radicals, modified saccharide radicals,
R₃ -H, -OH, -Oalkyl, -Oacyl, modified -Oacyl, saccharide radicals, modified saccharide radicals,
R₄, R₅ -COOH, carboxylic acid ester
as an antioxidant.

20. Compound of formula I 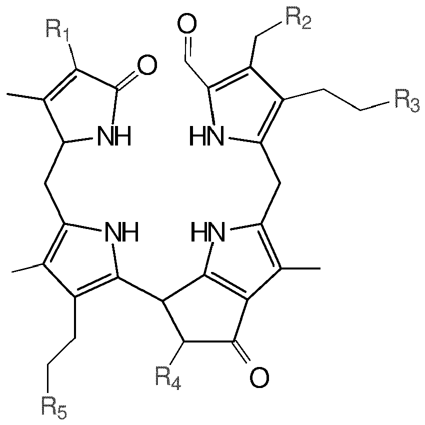 or a pharmacologically, dermatologically or cosmetically acceptable salt or derivative thereof, wherein R₁ to R₅ is selected from the radicals
R₁ -alkyl, -vinyl, -CHOH-CH₂OH
R₂ -H, -OH, -Oalkyl, -Oacyl, saccharide radicals, modified saccharide radicals,
R₃ -H, -OH, -Oalkyl, -Oacyl, modified -Oacyl, saccharide radicals, modified saccharide radicals,
R₄, R₅ -COOH, carboxylic acid ester
as a preservative.

21. Compound of formula I 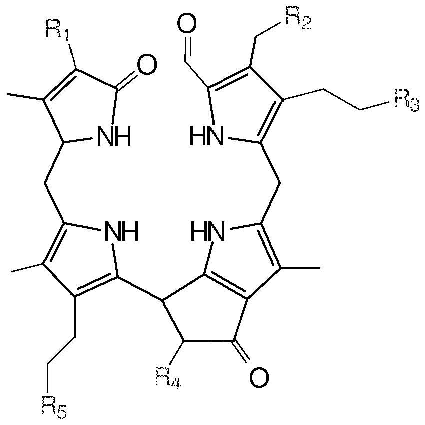 or a pharmacologically, dermatologically or cosmetically acceptable salt or derivative thereof, wherein R₁ to R₅ is selected from the radicals
R₁ -alkyl, -vinyl, -CHOH-CH₂OH
R₂ -H, -OH, -Oalkyl, -Oacyl, saccharide radicals, modified saccharide radicals,
R₃ -H, -OH, -Oalkyl, -Oacyl, modified -Oacyl, saccharide radicals, modified saccharide radicals,
R₄, R₅ -COOH, carboxylic acid ester
as an antibacterial agent.

22. Compound of formula I 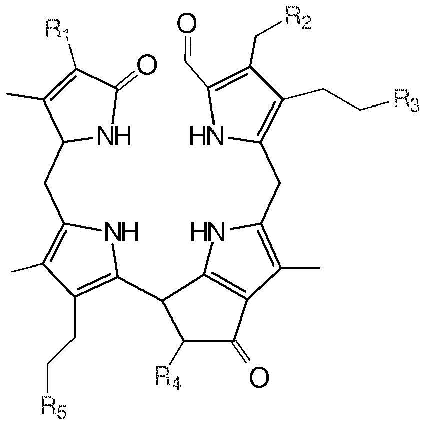 or a pharmacologically, dermatologically or cosmetically acceptable salt or derivative thereof, wherein R₁ to R₅ is selected from the radicals
R₁ -alkyl, -vinyl, -CHOH-CH₂OH
R₂ -H, -OH, -Oalkyl, -Oacyl, saccharide radicals, modified saccharide radicals,
R₃ -H, -OH, -Oalkyl, -Oacyl, modified -Oacyl, saccharide radicals, modified saccharide radicals,
R₄, R₅ -COOH, carboxylic acid ester
as a fungicide.

23. Compound of formula I 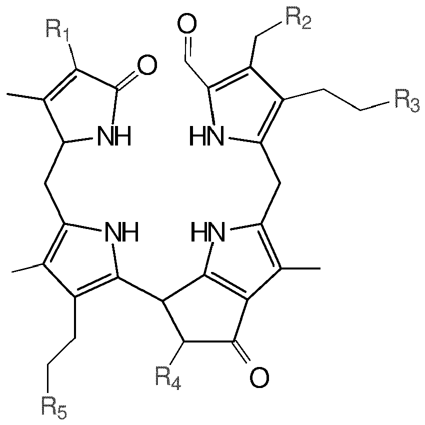 or a pharmacologically, dermatologically or cosmetically acceptable salt or derivative thereof, wherein R₁ to R₅ is selected from the radicals
R₁ -alkyl, -vinyl, -CHOH-CH₂OH
R₂ -H, -OH, -Oalkyl, -Oacyl, saccharide radicals, modified saccharide radicals,
R₃ -H, -OH, -Oalkyl, -Oacyl, modified -Oacyl, saccharide radicals, modified saccharide radicals,
R₄, R₅ -COOH, carboxylic acid ester
as a food supplement.

24. Use of a compound of formula I 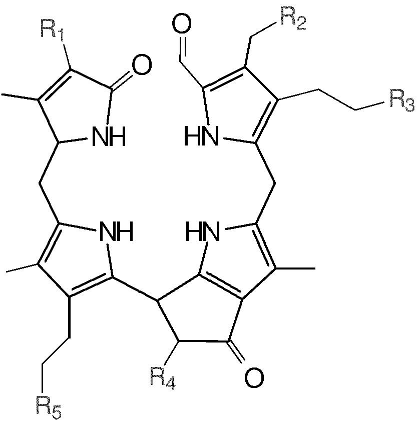 or a pharmacologically, dermatologically or cosmetically acceptable salt or derivative thereof, wherein R₁ to R₅ is selected from the radicals
R₁ -alkyl, -vinyl, -CHOH-CH₂OH
R₂ -H, -OH, -Oalkyl, -Oacyl, saccharide radicals, modified saccharide radicals,
R₃ -H, -OH, -Oalkyl, -Oacyl, modified -Oacyl, saccharide radicals, modified saccharide radicals,
R₄, R₅ -COOH, carboxylic acid ester
for the preparation of a medicinal product for the prevention and/or treatment of cancers.

25. Use according to claim 24, wherein the cancer comes from the group skin cancer, breast cancer, lung cancer, intestinal cancer or leukemia.

26. Use of a compound of formula I 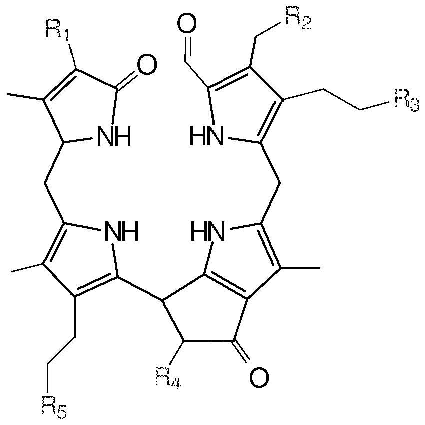 or a pharmacologically, dermatologically or cosmetically acceptable salt or derivative thereof, wherein R₁ to R₅ is selected from the radicals
R₁ -alkyl, -vinyl, -CHOH-CH₂OH
R₂ -H, -OH, -Oalkyl, -Oacyl, saccharide radicals, modified saccharide radicals,
R₃ -H, -OH, -Oalkyl, -Oacyl, modified -Oacyl, saccharide radicals, modified saccharide radicals,
R₄, R₅ -COOH, carboxylic acid ester
for the preparation of a cosmetic preparation, a pharmaceutical preparation, a foodstuff and/or a food supplement.

27. Use according to claim 26 for protection against oxidative stress and to combat allergies, inflammations and/or irritations.

28. Use according to claim 26 for the preparation of a preservative.

29. Process for obtaining non-fluorescent chlorophyll catabolites with the chemical formula I 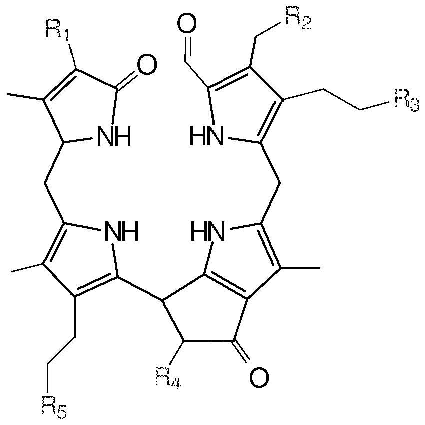 or a pharmacologically, dermatologically or cosmetically acceptable salt or derivative thereof, wherein R₁ to R₅ is selected from the radicals
R₁ -alkyl, -vinyl, -CHOH-CH₂OH
R₂ -H, -OH, -Oalkyl, -Oacyl, saccharide radicals, modified saccharide radicals
R₃ -H, -OH, -Oalkyl, -Oacyl, modified -Oacyl, saccharide radicals, modified saccharide radicals
R₄, R₅ -COOH, carboxylic acid ester
and also its salts and solvates from plant material, **characterized by** the steps:
- extraction of the plant material with a first solvent or solvent mixture with a dielectric constant of 5 to 20 and a dipole moment of 5 to 10 x 10⁻³⁰ Cm to form a crude extract
- filtration or chromatography of this extract over silica gel
- elution of the silica gel with a second solvent or solvent mixture, preferably with a greater polarity than the first solvent
- drawing off of the solvent and optional crystallization of the obtained residue.

30. Process according to claim 29, wherein CH₂Cl₂, methyl acetate, ethyl acetate, t-butanol, ethyl methyl ketone or mixtures thereof are used as the first solvent.

31. Process according to claim 29 or claim 30, wherein the filtration step is carried out with a silica-gel-filled chromatography column.

32. Process according to one of claims 29 to 31, wherein a solvent of greater polarity than the first solvent is used as solvent for the elution step and is taken from the group acetone, ethyl methyl ketone, methanol, methanol-water mixtures, methyl acetate or mixtures thereof or mixtures of the first solvent or solvent mixture with a solvent of greater polarity.

33. Process according to one of claims 29 to 32, wherein a purification step of the crude extract with a non-miscible solvent or a non-miscible aqueous solution takes place before the filtration step.

34. Process according to one of claims 29 to 33, wherein the eluate is washed with an aqueous solution.

35. Process according to one of claims 29 to 34, wherein the residue from the evaporation step is crystallized and then filtered and dried.

36. Process according to one of claims 29 to 35, wherein the silica gel has a particle size of less than 5,000.

37. Process according to one of claims 29 to 36, wherein plant parts which are green before ripening or senescence or contain chlorophylls are used as plant material.

38. Process according to one of claims 29 to 37, wherein substantially senescent or ripened plant material is used as plant material.

39. Process according to one of claims 29 to 38, wherein freshly harvested, senescent or ripened plant material, or senescent or ripened plant material which has been stored frozen after harvest (at approximately -20 °C or below), is used as plant material.

40. Process according to one of claims 29 to 39, wherein the plant material comes from agricultural plants such as oil seed rape, clover, barley, corn, tobacco, from herbaceous plants and agriculturally produced vegetables such as spinach, broccoli, pepper, from shrubs and broad-leaved trees such as witch hazel, elms, sycamores, walnuts, *Cercidiphyllum japonicum* and from conifers such as larches.

## Revendications

1. Préparation contenant au moins un composé de formule I 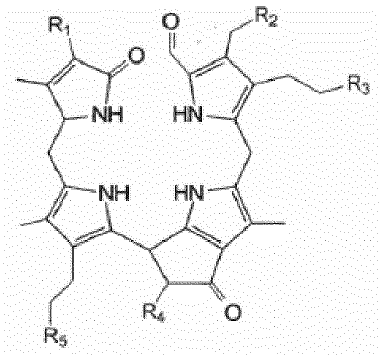 ou un de ses sels ou dérivés pharmacologiquement, dermatologiquement ou cosmétiquement compatibles, R₁ à R₅ étant sélectionnés parmi les groupes :
R₁ alkyle, vinyle, CHOH-CH₂OH
R₂ -H, -OH, -O-alkyle, -O-alcyle, groupe saccharide, groupe saccharide modifié,
R₃ -H, -OH, -O-alkyle, -O-acyle, -O-acyle modifié, groupe saccharide, groupe saccharide modifié,
R₄, R₅ -COOH, ester d'acide carboxylique,
ainsi qu'au moins un autre antioxydant et/ou une vitamine.

2. Préparation selon la revendication 1, **caractérisée en ce que** R₁ représente le vinyle (-CH=CH₂) ou -CHOH-CH₂OH, R₂ représente -H ou -OH, R₃ représente -H, -OH ou un groupe saccharide, R₄ représente -COOH ou -COOCH₃ et R₅ représente -COOH.

3. Préparation selon l'une des revendications 1 ou 2, **caractérisée en ce que** la préparation contient au moins un composé de formule I en une quantité supérieure à 0,001 %.

4. Préparation selon l'une des revendications 1 à 3, **caractérisée en ce que** les antioxydants et/ou les vitamines sont sélectionnés dans l'ensemble constitué de la vitamine A, du palmitate de vitamine A, de la vitamine C et de ses dérivés, du tocophérol, de l'acétate de tocophérol, de l'acide nicotinique, de l'acide pantothénique et de la biotine.

5. Préparation selon l'une des revendications 1 à 4, **caractérisée en ce que** la préparation contient un ou plusieurs filtres d'UV.

6. Préparation selon l'une des revendications 1 à 5, **caractérisée en ce qu'**elle est un agent cosmétique.

7. Préparation selon l'une des revendications 1 à 5, **caractérisée en ce qu'**elle est un médicament.

8. Préparation selon l'une des revendications 1 à 5, **caractérisée en ce qu'**elle est un agent de traitement de la peau.

9. Préparation selon l'une des revendications 1 à 8, **caractérisée en ce qu'**elle contient un véhicule compatible avec la peau et/ou une ou plusieurs substances actives à effet de soin de la peau et/ou anti-inflammatoire.

10. Préparation selon l'une des revendications 7 à 9, **caractérisée en ce qu'**elle se présente sous forme galénique.

11. Préparation selon l'une des revendications 1 à 4, **caractérisée en ce qu'**elle est un complément alimentaire.

12. Préparation selon l'une des revendications 1 à 4, **caractérisée en ce qu'**elle est un agent de conservation.

13. Préparation selon l'une des revendications 1 à 5 et 7 à 10, pour une utilisation comme médicament.

14. Préparation selon l'une des revendications 1 à 4, pour une utilisation comme agent antioxydant.

15. Utilisation d'une préparation selon l'une des revendications 1 à 5 comme agent de conservation.

16. Utilisation d'une préparation selon l'une des revendications 1 à 4 et 11 à 12, comme complément alimentaire.

17. Préparation selon l'une des revendications 1 à 5 ou 9 et 10, pour une utilisation comme agent dermatologique.

18. Composé de formule I 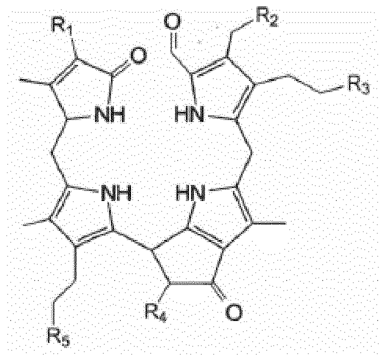 ou un de ses sels ou dérivés pharmacologiquement, dermatologiquement ou cosmétiquement compatibles, R₁ à R₅ étant sélectionnés parmi les groupes :
R₁ alkyle, vinyle, -CHOH-CH₂OH
R₂ -H, -OH, -O-alkyle, -O-acyle, groupe saccharide, groupe saccharide modifié,
R₃ -H, -OH, -O-alkyle, -O-acyle, -O-acyle modifié, groupe saccharide, groupe saccharide modifié,
R₄, R₅ -COOH, ester d'acide carboxylique,
pour une utilisation comme médicament.

19. Composé de formule I 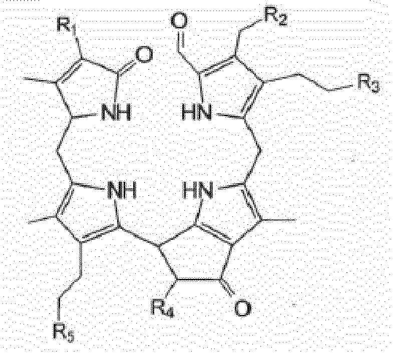 ou un de ses sels ou dérivés pharmacologiquement, dermatologiquement ou cosmétiquement compatibles, R₁ à R₅ étant sélectionnés parmi les groupes :
R₁ alkyle, vinyle, -CHOH-CH₂OH
R₂ -H, -OH, -O-alkyle, -O-alcyle, groupe saccharide, groupe saccharide modifié,
R₃ -H, -OH, -O-alkyle, -O-acyle, -O-acyle modifié, groupe saccharide, groupe saccharide modifié,
R₄, R₅ -COOH, ester d'acide carboxylique,
pour une utilisation comme agent antioxydant.

20. Composé de formule I 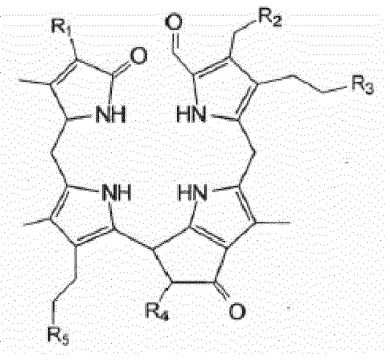 ou un de ses sels ou dérivés pharmacologiquement, dermatologiquement ou cosmétiquement compatibles, R₁ à R₅ étant sélectionnés parmi les groupes :
R₁ alkyle, vinyle, -CHOH-CH₂OH
R₂ -H, -OH, -O-alkyle, -O-alcyle, groupe saccharide, groupe saccharide modifié,
R₃ -H, -OH, -O-alkyle, -O-acyle, -O-acyle modifié, groupe saccharide, groupe saccharide modifié,
R₄, R₅ -COOH, ester d'acide carboxylique,
pour une utilisation comme agent de conservation.

21. Composé de formule I 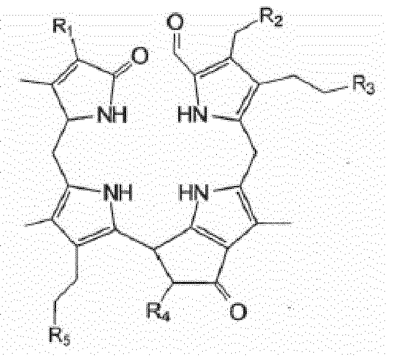 ou un de ses sels ou dérivés pharmacologiquement, dermatologiquement ou cosmétiquement compatibles, R₁ à R₅ étant sélectionnés parmi les groupes :
R₁ alkyle, vinyle, CHOH-CH₂OH
R₂ -H, -OH, -O-alkyle, -O-alcyle, groupe saccharide, groupe saccharide modifié,
R₃ -H, -OH, -O-alkyle, -O-acyle, -O-acyle modifié, groupe saccharide, groupe saccharide modifié,
R₄, R₅ -COOH, ester d'acide carboxylique,
pour une utilisation comme agent antibactérien.

22. Composé de formule I 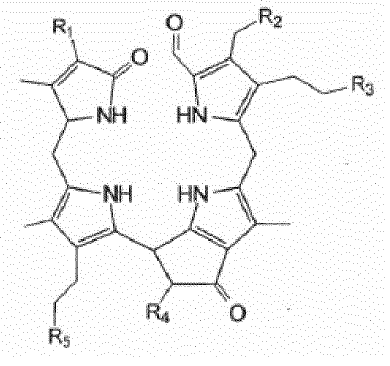 ou un de ses sels ou dérivés pharmacologiquement, dermatologiquement ou cosmétiquement compatibles, R₁ à R₅ étant sélectionnés parmi les groupes :
R₁ alkyle, vinyle, CHOH-CH₂OH
R₂ -H, -OH, -O-alkyle, -O-alcyle, groupe saccharide, groupe saccharide modifié,
R₃ -H, -OH, -O-alkyle, -O-acyle, -O-acyle modifié, groupe saccharide, groupe saccharide modifié,
R₄, R₅ -COOH, ester d'acide carboxylique,
pour une utilisation comme agent fongicide.

23. Composé de formule I 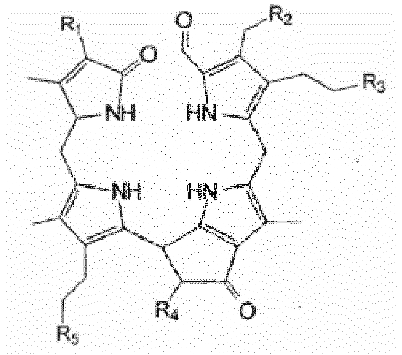 ou un de ses sels ou dérivés pharmacologiquement, dermatologiquement ou cosmétiquement compatibles, R₁ à R₅ étant sélectionnés parmi les groupes :
R₁ alkyle, vinyle, -CHOH-CH₂OH
R₂ -H, -OH, -O-alkyle, -O-alcyle, groupe saccharide, groupe saccharide modifié,
R₃ -H, -OH, -O-alkyle, -O-acyle, -O-acyle modifié, groupe saccharide, groupe saccharide modifié,
R₄, R₅ -COOH, ester d'acide carboxylique,
pour une utilisation comme complément alimentaire.

24. Composé de formule I 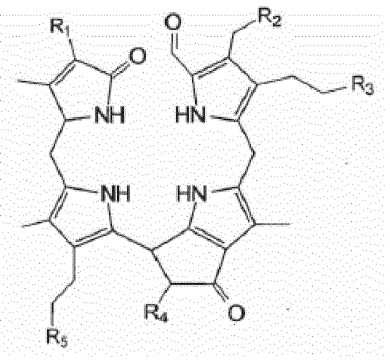 ou un de ses sels ou dérivés pharmacologiquement, dermatologiquement ou cosmétiquement compatibles, R₁ à R₅ étant sélectionnés parmi les groupes:
R₁ alkyle, vinyle, CHOH-CH₂OH
R₂ -H, -OH, -O-alkyle, -O-alcyle, groupe saccharide, groupe saccharide modifié,
R₃ -H, -OH, -O-alkyle, -O-acyle, -O-acyle modifié, groupe saccharide, groupe saccharide modifié,
R₄, R₅ -COOH, ester d'acide carboxylique,
pour la préparation d'un médicament destiné à prévenir et/ou traiter des cancers.

25. Utilisation selon la revendication 24, **caractérisée en ce que** le cancer fait partie de l'ensemble des cancers de la peau, des cancers du sein, des cancers du poumon, des cancers de l'intestin et de la leucémie.

26. Composé de formule I 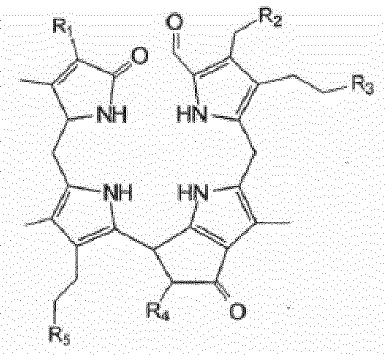 ou un de ses sels ou dérivés pharmacologiquement, dermatologiquement ou cosmétiquement compatibles, R₁ à R₅ étant sélectionnés parmi les groupes :
R₁ alkyle, vinyle, -CHOH-CH₂OH
R₂ -H, -OH, -O-alkyle, -O-alcyle, groupe saccharide, groupe saccharide modifié,
R₃ -H, -OH, -O-alkyle, -O-acyle, -O-acyle modifié, groupe saccharide, groupe saccharide modifié,
R₄, R₅ -COOH, ester d'acide carboxylique,
pour la préparation d'une préparation cosmétique, d'une préparation pharmaceutique, d'un aliment et/ou d'un complément alimentaire.

27. Utilisation selon la revendication 26 dans la protection contre le stress oxydatif et la lutte contre les allergies, les inflammations et/ou les irritations.

28. Utilisation selon la revendication 26 en vue de la préparation d'un agent de conservation.

29. Procédé d'obtention de métabolites non fluorescents de la chlorophylle de formule chimique I 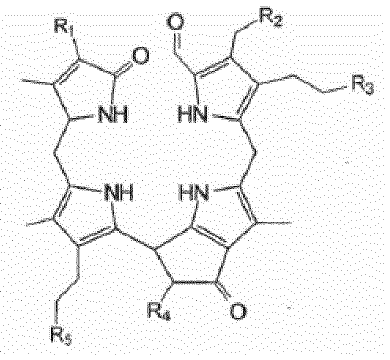 ou d'un de ses sels ou dérivés pharmacologiquement, dermatologiquement ou cosmétiquement compatibles, R₁ à R₅ étant sélectionnés parmi les groupes :
R₁ alkyle, vinyle, -CHOH-CH₂OH
R₂ -H, -OH, -O-alkyle, -O-alcyle, groupe saccharide, groupe saccharide modifié,
R₃ -H, -OH, -O-alkyle, -O-acyle, -O-acyle modifié, groupe saccharide, groupe saccharide modifié,
R₄, R₅ -COOH, ester d'acide carboxylique,
ainsi que de ses sels et solvates provenant de matériaux végétaux, le procédé étant **caractérisé par** les étapes qui consistent à :
- extraire le matériau végétal à l'aide d'un premier solvant ou mélange de solvants dont la constante diélectrique est de 5 à 20 et le moment dipolaire de 5 à 10 x 10⁻³⁰ cm pour obtenir un extrait brut,
- filtration ou chromatographie de cet extrait sur gel de silice,
- élution du gel de silice à l'aide d'un deuxième solvant ou mélange de solvants dont la polarité est plus élevée que celle du premier solvant et
- extraction du solvant et éventuellement cristallisation du résidu ainsi obtenu.

30. Procédé selon la revendication 29, **caractérisé en ce qu'**il utilise comme premier solvant CH₂Cl₂, l'acétate de méthyle, l'acétate d'éthyle, le t-butanol, l'éthylméthylcétone ou leurs mélanges.

31. Procédé selon les revendications 29 ou 30, **caractérisé en ce que** l'étape de filtration est exécutée sur une colonne de chromatographie remplie de gel de silice.

32. Procédé selon l'une des revendications 29 à 31, **caractérisé en ce que** le solvant de l'étape d'élution est un solvant dont la polarité est supérieure à celle du premier solvant et est sélectionné dans l'ensemble constitué de l'acétone, de l'éthylméthylcétone, du méthanol, des mélanges de méthanol et d'eau, de l'acétate de méthyle ou de leurs mélanges ou de mélanges du premier solvant ou mélanges de solvants avec un solvant de plus grande polarité.

33. Procédé selon l'une des revendications 29 à 32, **caractérisé en ce qu'**une étape de purification de l'extrait brut à l'aide d'un solvant non miscible ou d'une solution aqueuse non miscible est réalisée avant l'étape de filtration.

34. Procédé selon l'une des revendications 29 à 33, **caractérisé en ce que** l'éluat est lavé à l'aide d'une solution aqueuse.

35. Procédé selon l'une des revendications 29 à 34, **caractérisé en ce que** le résidu de l'étape de vaporisation est cristallisé et ensuite filtré et séché.

36. Procédé selon l'une des revendications 29 à 35, **caractérisé en ce que** le gel de silice présente une granulométrie inférieure à 5 000.

37. Procédé selon l'une des revendications 29 à 36, **caractérisé en ce que** comme matériau végétal, il utilise des parties de plantes vertes avant leur maturation ou leur sénescence.

38. Procédé selon l'une des revendications 29 à 37, **caractérisé en ce que** comme matériau végétal, il utilise un matériau végétal essentiellement vieilli ou maturé.

39. Procédé selon l'une des revendications 29 à 38, **caractérisé en ce que** comme matériau végétal, il utilise un matériau végétal fraichement récolté, vieilli ou maturé ou un matériau végétal vieilli ou maturé qui, après la récolte, a été entreposé dans des conditions de surgélation (à environ -20°C ou moins).

40. Procédé selon l'une des revendications 29 à 39, **caractérisé en ce que** le matériau végétal provient de plantes cultivées telles que le colza, le trèfle, l'orge, le maïs, le tabac, des graminées et des légumes produits en agriculture, par exemple l'épinard, le brocoli, le paprika, des arbustes et des feuillus tels que l'hamamélis, l'orme, le platane, le noyer, le *cercidiphyllum japonicum* et des résineux, par exemple le mélèze.
